(19) Europäisches Patentamt / European Patent Office / Office européen des brevets

(11) **EP 1 495 142 B1**

(12) **EUROPEAN PATENT SPECIFICATION**

(45) Date of publication and mention of the grant of the patent:
**26.09.2007 Bulletin 2007/39**

(51) Int Cl.:
*C12N 15/63* (2006.01)   *C12N 15/10* (2006.01)
*C07K 16/18* (2006.01)   *C12Q 1/68* (2006.01)
*C12P 21/00* (2006.01)

(21) Application number: **03726328.2**

(22) Date of filing: **16.04.2003**

(86) International application number:
**PCT/US2003/011935**

(87) International publication number:
**WO 2003/089671 (30.10.2003 Gazette 2003/44)**

(54) **"DOPING" IN WALK-THROUGH MUTAGENESIS**

DOPING BEI DER WALK-THROUGH-MUTAGENESE

DOPAGE   DANS LA MUTAGENESE TRAVERSANTE

(84) Designated Contracting States:
**AT BE BG CH CY CZ DE DK EE ES FI FR GB GR HU IE IT LI LU MC NL PT RO SE SI SK TR**

(30) Priority: **17.04.2002 US 373686 P**

(43) Date of publication of application:
**12.01.2005 Bulletin 2005/02**

(73) Proprietors:
• **Bioren, Inc.**
  **San Carlos, CA 94070 (US)**
• **Cappuccilli, Guido**
  **San Mateo, CA 94402 (US)**

(72) Inventors:
• **Crea, Roberto**
  **San Mateo, CA 94402 (US)**
• **Cappuccilli, Guido**
  **San Mateo, CA 94402 (US)**

(74) Representative: **Kirkham, Nicholas Andrew et al**
Graham Watt & Co LLP
St Botolph's House
7-9 St Botolph's Road
Sevenoaks
Kent TN13 3AJ (GB)

(56) References cited:
WO-A-01/48485          WO-A-91/15581
WO-A-97/30150          US-A- 5 798 208
US-A- 5 830 650        US-B1- 6 432 675

• WEISS G A ET AL: "Rapid mapping of protein functional epitopes by combinatorial alanine scanning." PROCEEDINGS OF THE NATIONAL ACADEMY OF SCIENCES OF THE UNITED STATES OF AMERICA. 1 AUG 2000, vol. 97, no. 16, 1 August 2000 (2000-08-01), pages 8950-8954, XP002161102 ISSN: 0027-8424
• ARKIN ET AL.: 'Optimizing nucleotide mixtures to encode specific subsets of amino acids for semi-random mutagenesis' BIO/TECHNOLOGY vol. 10, March 1992, pages 297 - 300, XP000942164
• DUBNICK ET AL.: 'Mixed oligo-designer (MOD), a computer program to aid planning of automated, mixed oligodeoxyribonucleotide synthesis for mutagenesis experiments' GENE vol. 105, 1991, pages 1 - 7, XP002973145
• JENSEN ET AL: "Scoring functions for computational algorithms applicable to the design of spiked oligonucleotides" NUCLEIC ACIDS RESEARCH, vol. 26, no. 3, 1998, pages 697-702,
• HERMES ET AL: "A reliable method for random mutagensis: the generation of mutant libraries using spiked oligodeoxyribonucleotide primers" GENE, vol. 84, 1989, pages 143-151,

SEQUENCE LISTING

<110> Crea, Roberto
cappuccilli, Guido

<120> "DOPING" IN WALK-THROUGH MUTAGENESIS

<130> 2345.2002003

<140> PCT/US03/11935
<141> 2003-04-16

<150> 60/373,686<151> 2002-04-17

<160> 86

<170> FastSEQ for windows version 4.0

<210> 1
<211> 5
<212> PRT
<213> Artificial Sequence

<220>
<223> peptide

<400> 1

```
Asp Phe Tyr Met Gly
 1               5
```

<210> 2
<211> 14
<212> PRT
<213> Artificial Sequence

<220>
<223> peptide

<400> 2

```
Gly Asn Lys Tyr Thr Thr Glu Tyr Ser Ala Ser Val Lys Gly
 1           5                   10
```

<210> 3
<211> 11
<212> PRT
<213> Artificial sequence

<220>
<223> peptide

<400> 3

```
Asn Tyr Tyr Gly Ser Thr Trp Tyr Phe Asp Val
 1           5                   10
```

<210> 4
<211> 15
<212> DNA
<213> Artificial sequence

<220>
<223> oligonucleotide

<400> 4
gacttctaca tggag            15

<210> 5
<211> 15
<212> DNA
<213> Artificial sequence

<220>
<223> oligonucleotide

<400> 5
gackwckacr wkgak          15

<210> 6
<211> 5
<212> PRT
<213> Artificial sequence

<220>
<223> peptide

<223> At Position 2, Xaa = PHE, ASP, TYR or VAL

<223> At Position 3, Xaa = TYR or ASP

<223> At Position 4, Xaa = MET, ASP, VAL, GLU, ASN, ILE
or LYS

<223> At Position 5, Xaa = GLU or ASP

<221> VARIANT
<222> (1)...(5)
<223> Xaa = Any Amino Acid

<400> 6

```
        Asp Xaa Xaa Xaa Xaa
         1                5
```

<210> 7
<211> 42
<212> DNA
<213> Artificial Sequence

<220>
<223> oligonucleotide

<400> 7
ggtaacaagt atactactga atacagcgct tctgttaaag gt          42

<210> 8
<211> 42
<212> DNA
<213> Artificial sequence

<220>
<223> oligonucleotide

<400> 8

srtmacmaky atmmtmmtsa kyacmrcsrt ymtswtmams rt          42

<210> 9
<211> 14
<212> PRT
<213> Artificial Sequence


<220>
<223> peptide
<223> At Position 1, Xaa = GLY, HIS, ARG or ASP
<223> At Position 2, Xaa = ASN or HIS
<223> At Position 3, Xaa = LYS, HIS, ASN or GLY
<223> At Position 4, Xaa = TYR or HIS
<223> At Poistion 5, Xaa = THR, HIS, ASN or PRO
<223> At Position 6, Xaa = THR, HIS, ASN or PRO
<223> At Position 7, Xaa = GLU, HIS, ASP or GLN
<223> At Position 8, Xaa = TyR or HIS
<223> At Position 9, Xaa = SER, HIS, ARG or ASN
<223> At Position 10, Xaa = ALA, HIS, ASP or PRO
<223> At Position 11, Xaa = SER, HIS, PRO or TYR
<223> At Position 12, Xaa = VAL, HIS, ASP or LEU
<223> At Position 13, Xaa = LYS, HIS, ASN or GLN
<223> At Position 14, Xaa = GLY, HIS ARG or ASP


<221> VARIANT
<222> (1)...(14)
<223> xaa = Any Amino Acid


<400> 9


```
        Xaa Xaa Xaa Xaa Xaa Xaa Xaa Xaa Xaa Xaa Xaa Xaa Xaa Xaa
         1                   5                   10
```


<210> 10
<211> 33
<212> DNA
<213> Artificial Sequence


<220>
<223> oligonucleotide


<400> 10
aactactatg gcagcacttg gtacttcgac ggt          33


<210> 11
<211> 33
<212> DNA
<213> Artificial sequence


<220>
<223> oligonucleotide


<400> 11
arctmctmtr cgacgastts gtmctyckmc kyt          33


<210> 12
<211> 11

<212> PRT
<213> Artificial Sequence

<220>
<223> peptide
<223> At Position 1, Xaa = ASN or SER
<223> At Position 2, Xaa = TYR or SER
<223> At Position 3, Xaa = TYR or SER
<223> At Position 4, Xaa = GLY or SER
<223> At Position 6, Xaa = THR or SER
<223> At Position 7, Xaa = TRP or SER
<223> At position 8, Xaa = TYR or SER
<223> At Position 9, Xaa = PHE or SER
<223> At Position 10, Xaa = ASP, SER, ALA or TYR
<223> At Position 11, Xaa = VAL, SER, ALA or PHE

<221> VARIANT
<222> (1)...(11)
<223> xaa = Any Amino Acid

<400> 12

```
Xaa Xaa Xaa Xaa Ser Xaa Xaa Xaa Xaa Xaa Xaa
 1               5               10
```

<210> 13
<211> 5
<212> PRT
<213> Artificial Sequence

<220>
<223> peptide

<400> 13

```
Asp Tyr Tyr Met Glu
 1               5
```

<210> 14
<211> 5
<212> PRT
<213> Artificial Sequence

<220>
<223> peptide

<400> 14

```
Asp Val Tyr Met Glu
 1               5
```

<210> 15
<211> 5
<212> PRT
<213> Artificial Sequence

<220>
<223> peptide

<400> 15

Asp Asp Asp Ile Glu
1               5

<210> 16
<211> 5
<212> PRT
<213> Artificial sequence

<220>
<223> peptide

<400> 16

Asp Asp Asp Glu Asp
1               5

<210> 17
<211> 5
<212> PRT
<213> Artificial sequence

<220>
<223> peptide

<400> 17

Asp Val Asp Asp Asp
1               5

<210> 18
<211> 5
<212> PRT
<213> Artificial sequence

<220>
<223> peptide

<400> 18

Asp Phe Asp Met Asp
1               5

<210> 19
<211> 5
<212> PRT
<213> Artificial Sequence

<220>
<223> peptide

<400> 19

```
            Asp Tyr Tyr Val Glu
            1               5
```

<210> 20
<211> 5
<212> PRT
<213> Artificial sequence

<220>
<223> peptide

<400> 20

```
            Asp Tyr Asp Val Asp
            1               5
```

<210> 21
<211> 5
<212> PRT
<213> Artificial sequence

<220>
<223> peptide

<400> 21

```
            Asp Asp Asp Val Glu
            1               5
```

<210> 22
<211> 5
<212> PRT
<213> Artificial sequence

<220>
<223> peptide

<400> 22

```
            Asp Phe Asp Lys Asp
            1               5
```

<210> 23
<211> 5
<212> PRT
<213> Artificial sequence

<220>
<223> peptide

<400> 23

```
                    Asp Val Asp Asp Glu
                     1               5
```

<210> 24
<211> 5
<212> PRT
<213> Artificial sequence

<220>
<223> peptide

<400> 24

```
                    Asp Asp Asp Lys Asp
                     1               5
```

<210> 25
<211> 5
<212> PRT
<213> Artificial sequence

<220>
<223> peptide

<400> 25

```
                    Asp Val Asp Met Asp
                     1               5
```

<210> 26
<211> 5
<212> PRT
<213> Artificial sequence

<220>
<223> peptide

<400> 26

```
                    Asp Asp Asp Asp Glu
                     1               5
```

<210> 27
<211> 5
<212> PRT
<213> Artificial sequence

<220>
<223> peptide

<400> 27

```
                    Asp Val Asp Asp Glu
                     1               5
```

Asp Phe Asp Val Asp
1 5

<210> 28
<211> 5
<212> PRT
<213> Artificial sequence

<220>
<223> peptide

<400> 28

Asp Tyr Asp Asp Asp
1 5

<210> 29
<211> 5
<212> PRT
<213> Artificial Sequence

<220>
<223> peptide

<400> 29

Asp Val Tyr Lys Glu
1 5

<210> 30
<211> 5
<212> PRT
<213> Artificial sequence

<220>
<223> peptide

<400> 30

Asp Phe Asp Val Asp
1 5

<210> 31
<211> 14
<212> PRT
<213> Artificial Sequence

<220>
<223> peptide

<400> 31

Arg Asn His Asn Pro Thr Glu Tyr His His Ser Val Gln Asp
1               5                   10

<210> 32
<211> 14
<212> PRT
<213> Artificial sequence

<220>
<223> peptide

<400> 32

Arg His His His His Thr Gln His Arg Ala Ser Asp Asn Gly
1               5                   10

<210> 33
<211> 14
<212> PRT
<213> Artificial sequence

<220>
<223> peptide

<400> 33

Gly Asn His His Pro Asn His His Asn Pro Ser Val His His
1               5                   10

<210> 34
<211> 16
<212> PRT
<213> Artificial sequence

<220>
<223> peptide

<400> 34

Arg Asn Lys Tyr Pro Asn Asp Tyr Ala Arg Thr Pro Ser Asp Asn Gly
1               5                   10                  15

<210> 35
<211> 14
<212> PRT
<213> Artificial sequence

<220>
<223> peptide

<400> 35

Arg His Asn His Pro Pro Gln Tyr His Pro Ser Leu His His
1                   5                   10

<210> 36
<211> 14
<212> PRT
<213> Artificial sequence

<220>
<223> peptide

<400> 36

Arg His Lys His Pro Thr Gln His His Asp Tyr Val Lys Arg
1                   5                   10

<210> 37
<211> 15
<212> PRT
<213> Artificial Sequence

<220>
<223> peptide

<400> 37

His His Arg His Pro Thr His His Asn Pro Pro His Asp His His
1                   5                   10                   15

<210> 38
<211> 14
<212> PRT
<213> Artificial Sequence

<220>
<223> peptide

<400> 38

Arg His Lys Tyr Thr Pro Asp His Arg His His Leu Gln Asp
1                   5                   10

<210> 39
<211> 15
<212> PRT
<213> Artificial Sequence

<220>
<223> peptide

<400> 39

```
Gly Asn Lys His Pro Pro His Glu His Asn Pro Pro Asp Lys His
 1               5               10                      15
```

<210> 40
<211> 14
<212> PRT
<213> Artificial sequence

<220>
<223> peptide

<400> 40

```
Gly Asn Gln Tyr His His His Tyr Arg His Tyr Asp His Gly
 1               5               10
```

<210> 41
<211> 14
<212> PRT
<213> Artificial sequence

<220>
<223> peptide

<400> 41

```
Asp His His His Thr His Glu Tyr Ser Asp Tyr Val Asn Asp
 1               5               10
```

<210> 42
<211> 14
<212> PRT
<213> Artificial sequence

<220>
<223> peptide

<400> 42

```
Arg Asn Lys Tyr His Pro His Tyr Arg His Ser Leu Asn Arg
 1               5               10
```

<210> 43
<211> 14
<212> PRT
<213> Artificial Sequence

<220>
<223> peptide

<400> 43

```
His His His His His Pro Asp His Asn Asp His His Gln His
 1               5                  10
```

<210> 44
<211> 14
<212> PRT
<213> Artificial Sequence

<220>
<223> peptide

<400> 44

```
His His Gln Tyr His Thr His Tyr Asn Asp His Asp Asn Asp
 1               5                  10
```

<210> 45
<211> 14
<212> PRT
<213> Artificial Sequence

<220>
<223> peptide

<400> 45

```
Gly Asn Lys Tyr Thr His Asp His Ser Asp His Asp Lys Arg
 1               5                  10
```

<210> 46
<211> 14
<212> PRT
<213> Artificial Sequence

<220>
<223> peptide

<400> 46

```
Gly His Gln His His Pro Asp Tyr Ser Ala Pro Asp His His
 1               5                  10
```

<210> 47
<211> 14
<212> PRT
<213> Artificial sequence

<220>
<223> peptide

<400> 47

Arg Asn Asn His His Thr His Tyr Asn Asp Ser His Asn Arg
1                   5                   10

<210> 48
<211> 14
<212> PRT
<213> Artificial sequence

<220>
<223> peptide

<400> 48

Gly Asn Lys Tyr Thr Thr Gln Tyr Ser Ala Ser Val Lys Gly
1                   5                   10

<210> 49
<211> 11
<212> PRT
<213> Artificial Sequence

<220>
<223> peptide

<400> 49

Ser Ser Ser Gly Ser Thr Ser Tyr Ser Ser Ala
1                   5                   10

<210> 50
<211> 11
<212> PRT
<213> Artificial Sequence

<220>
<223> peptide

<400> 50

Ser Tyr Ser Gly Ser Thr Trp Tyr Phe Ser Val
1                   5                   10

<210> 51
<211> 11
<212> PRT
<213> Artificial Sequence

<220>
<223> peptide

<400> 51

```
Ser Tyr Ser Ser Ser Ser Trp Tyr Phe Ser Ala
 1               5               10
```

<210> 52
<211> 11
<212> PRT
<213> Artificial Sequence

<220>
<223> peptide

<400> 52

```
Ser Tyr Ser Gly Ser Ser Trp Ser Phe Asp Val
 1               5               10
```

<210> 53
<211> 11
<212> PRT
<213> Artificial sequence

<220>
<223> peptide

<400> 53

```
Asn Tyr Tyr Gly Ser Ser Trp Tyr Ser Ser Ala
 1               5               10
```

<210> 54
<211> 11
<212> PRT
<213> Artificial sequence

<220>
<223> peptide

<400> 54

```
Asn Ser Tyr Ser Ser Ser Ser Tyr Ser Tyr Ala
 1               5               10
```

<210> 55
<211> 11
<212> PRT
<213> Artificial Sequence

<220>
<223> peptide

<400> 55

```
Ser Tyr Ser Gly Ser Ser Trp Tyr Ser Asp Val
 1               5                   10
```

<210> 56
<211> 11
<212> PRT
<213> Artificial Sequence

<220>
<223> peptide

<400> 56

```
Ser Tyr Tyr Ser Ser Ser Trp Ser Ser Asp Ser
 1               5                   10
```

<210> 57
<211> 11
<212> PRT
<213> Artificial Sequence

<220>
<223> peptide

<400> 57

```
Asn Tyr Tyr Ser Ser Ser Trp Tyr Phe Tyr Ala

     1               5                   10
```

<210> 58
<211> 11
<212> PRT
<213> Artificial sequence

<220>
<223> peptide

<400> 58

```
Ser Tyr Ser Gly Ser Thr Ser Tyr Ser Ala Ala
 1               5                   10
```

<210> 59
<211> 11
<212> PRT
<213> Artificial Sequence

<220>
<223> peptide

<400> 59

```
                Ser Tyr Tyr Ser Ser Ser Trp Tyr Ser Tyr Ser
                  1               5               10
```

<210> 60
<211> 11
<212> PRT
<213> Artificial Sequence

<220>
<223> peptide

<400> 60

```
                Asn Ser Tyr Gly Ser Ser Trp Tyr Phe Ala Ala
                  1               5               10
```

<210> 61
<211> 11
<212> PRT
<213> Artificial sequence

<220>
<223> peptide

<400> 61

```
                Ser Tyr Ser Gly Ser Ser Trp Ser Ser Tyr Ala
                  1               5               10
```

<210> 62
<211> 11
<212> PRT
<213> Artificial sequence

<220>
<223> peptide

<400> 62

```
                Asn Tyr Ser Ser Ser Ser Trp Tyr Ser Asp Val
                  1               5               10
```

<210> 63
<211> 11
<212> PRT
<213> Artificial Sequence

<220>
<223> peptide

<400> 63

```
Asn Ser Ser Gly Ser Ser Ser Tyr Ser Tyr Val
 1               5               10
```

<210> 64
<211> 11
<212> PRT
<213> Artificial sequence

<220>
<223> peptide

<400> 64

```
Ser Ser Tyr Gly Ser Ser Ser Tyr Phe Ser Ser
 1               5               10
```

<210> 65
<211> 11
<212> PRT
<213> Artificial Sequence

<220>
<223> peptide

<400> 65

```
Ser Tyr Ser Ser Ser Ser Ser Tyr Ser Asp Val
 1               5               10
```

<210> 66
<211> 11
<212> PRT
<213> Artificial Sequence

<220>
<223> peptide

<400> 66

```
Asn Ser Ser Ser Ser Ser Trp Tyr Phe Asp Ala
 1               5               10
```

<210> 67
<211> 14
<212> PRT
<213> Artificial sequence

<220>
<223> peptide

<400> 67

Gly Asn Lys Tyr Thr Thr Glu Tyr Ser Ala Ser Val Lys Asp
1 5 10

<210> 68
<211> 14
<212> PRT
<213> Artificial Sequence

<220>
<223> peptide

<400> 68

Gly Asn Lys Tyr Thr Thr Asp Tyr Ser Ala Pro Asp Lys Gly
1 5 10

<210> 69
<211> 14
<212> PRT
<213> Artificial Sequence

<220>
<223> peptide

<400> 69

Gly Asn Lys Tyr Thr Thr Glu Tyr Arg Ala Ser Val Lys Gly
1 5 10

<210> 70
<211> 14
<212> PRT
<213> Artificial sequence

<220>
<223> peptide

<400> 70

Arg Asn Lys Tyr Asn Thr Glu Tyr Ser Ala Ser Val Lys Gly
1 5 10

<210> 71
<211> 14
<212> PRT
<213> Artificial Sequence

<220>
<223> peptide

<400> 71

Gly Asn Lys Tyr Thr Thr Glu Tyr Ser Asp Pro Val Lys Gly
1                   5                   10

<210> 72
<211> 14
<212> PRT
<213> Artificial Sequence

<220>
<223> peptide

<400> 72

Gly His Lys Tyr Thr Thr Glu Tyr Arg Asp Ser Val Arg Gly
1                   5                   10

<210> 73
<211> 14
<212> PRT
<213> Artificial Sequence

<220>
<223> peptide

<400> 73

Gly Asn Lys Tyr Asn Thr Glu Tyr Arg Ala Ser Leu Lys Arg
1                   5                   10

<210> 74
<211> 14
<212> PRT
<213> Artificial Sequence

<220>
<223> peptide

<400> 74

Arg Asn Lys Tyr Thr Pro Glu Tyr Ser Ala Ser Val Lys Gly
1                   5                   10

<210> 75
<211> 14
<212> PRT
<213> Artificial Sequence

<220>
<223> peptide

<400> 75

Asp Asn Asn Tyr Thr Thr Glu Tyr Asn Ala Ser Leu Lys Gly
1              5            10

<210> 76
<211> 14
<212> PRT
<213> Artificial sequence

<220>
<223> peptide

<400> 76

Asp Asn Asn Tyr Thr Thr Glu Tyr Ser Ala Ser Val Asn Gly
1              5            10

<210> 77
<211> 14
<212> PRT
<213> Artificial sequence

<220>
<223> peptide

<400> 77

Gly Asn Lys Tyr Thr Asn Gln Tyr Ser Pro Ser Asp Lys Gly
1              5            10

<210> 78
<211> 14
<212> PRT
<213> Artificial sequence

<220>
<223> peptide

<400> 78

Gly Asn Asn Tyr Thr Asn Glu Tyr Ser Ala Ser Val Lys Gly
1              5            10

<210> 79
<211> 14
<212> PRT
<213> Artificial Sequence

<220>
<223> peptide

<400> 79

```
        Gly Asn Lys Tyr Thr Thr Glu Tyr Ser Ala Ser Asp Lys Asp
        1                   5                   10
```

<210> 80
<211> 14
<212> PRT
<213> Artificial sequence

<220>
<223> peptide

<400> 80

```
        Asp Asn Asn His Pro Asn Glu Tyr Ser Pro Ser Val Asn Gly
        1                   5                   10
```

<210> 81
<211> 14
<212> PRT
<213> Artificial Sequence

<220>
<223> peptide

<400> 81

```
        Gly Asn Lys Tyr Thr Thr Glu Tyr Asn Pro Ser Asp Lys Gly
        1                   5                   10
```

<210> 82
<211> 14
<212> PRT
<213> Artificial Sequence

<220>
<223> peptide

<400> 82

```
        Asp Asn Lys Tyr Thr Thr Glu Tyr Ser Ala Ser Val Lys Gly
        1                   5                   10
```

<210> 83
<211> 14
<212> PRT
<213> Artificial Sequence

<220>
<223> peptide

<400> 83

```
Gly Asn Lys Tyr Thr Thr Glu Tyr Ser Ala Ser Val Gln Gly
 1               5               10
```

<210> 84
<211> 14
<212> PRT
<213> Artificial Sequence

<220>
<223> peptide

<400> 84

```
His Asn Lys Tyr Thr Thr His Tyr Ser Ala Ser Leu Lys Gly
 1               5               10
```

<210> 85
<211> 14
<212> PRT
<213> Artificial Sequence

<220>
<223> peptide

<400> 85

```
Gly Asn Lys Tyr Thr Asn Glu Tyr Asn Ala Ser Asp Lys Arg
 1               5               10
```

<210> 86
<211> 14
<212> PRT
<213> Artificial Sequence

<220>
<223> peptide

<400> 86

```
Gly Asn Lys Tyr Thr Thr Glu Tyr Arg Ala Ser Val Gln Asp
 1               5               10
```

**Description**

BACKGROUND OF THE INVENTION

Mutagenesis is a powerful tool in the study of protein structure and function. Mutations can be made in the nucleotide sequence of a cloned gene encoding a protein of interest and the modified gene can be expressed to produce mutants of the protein. By comparing the properties of a wild-type protein and the mutants generated, it is often possible to identify individual amino acids or domains of amino acids that are essential for the structural integrity and/or biochemical function of the protein, such as its binding and/or catalytic activity. The number of mutants that can be generated from a single protein, however, renders it difficult to select mutants that will be informative or have a desired property, even if the selected mutants which encompass mutations solely in specific, putatively important regions of a protein (e.g., regions at or around the active site of a protein). For example, the substitution, deletion or insertion of a particular amino acid may have a local or global effect on the protein. A need remains for a means to assess the effects of mutagenesis of a protein systematically.

WO91/15581 discloses a method of walk-through Mutagenesis.

G. Weiss et al disclose rapid mapping of protein functional epitopes in PNAS, August 1, 2000, vol 97 no. 16, 8950-8954.

J. Hermes et al disclose a reliable method for randon mutagenesis: the generation of mutant libraries using spiked oligodeoxyribonucleotide primers in Gene, 84 (1989) 143-151.

L. Jensen et al, disclose scoring functions for computational algorithms applicable to the design of spiked oligonucleotides in Nucleic Acids Research, 1998, vol 26, No. 3, 697-702.

WO 01/48485 discloses the use of a native epitope for selecting evolved binding members from a library of mutants of a protein capable of binding to said epitope.

SUMMARY OF THE INVENTION

The current invention pertains to methods of walk-through mutagenesis of a nucleic acid encoding a polypeptide of interest. In the methods, one or more target regions of amino acids in the wild-type (prototype) polypeptide of interest are selected; representative target regions include, for example, functional domains of the polypeptide, such as a hypervariable region of an antibody. For each target region, one predetermined amino acid to be incorporated into the target region in lieu of the prototype amino acids are selected. A mixture of oligonucleotides is synthesized, in which the oligonucleotides comprise a nucleotide sequence for each target region, and at each sequence position in the target region, contain either a nucleotide that is required for synthesis of the prototype amino acid of the polypeptide (a "prototype nucleotide"), or a nucleotide that is required for synthesis of the predetermined amino acid (a "predetermined nucleotide"). During synthesis, "doping" is used; "doping" indicates that the ratio of prototype nucleotides, to predetermined nucleotides, that are available to be incorporated into the oligonucleotides during the synthesis, is greater than 1:1, preferably 4:1 or greater than 4:1, even more preferably 7:1 or greater than 7:1, and still more preferably 9:1 or greater than 9:1. In one embodiment, the ratio of prototype nucleotides, to predetermined nucleotides, is determined using a binomial distribution that takes into consideration the length of the target region and a desired degree of success in incorporating nucleotides that encode the predetermined amino acid.

The methods of the invention allow production of mutant polypeptide in which the overall presence (walk-through) of the predetermined amino acid is limited to one or two positions per mutated polypeptide, leaving the remaining amino acids in the targeted region intact or as close as possible to the prototype sequence. In this way, more precise and specific chemical variations can be produced, quickly and in a systematic manner.

BRIEF DESCRIPTION OF THE DRAWINGS

FIG. 1 is a schematic depiction Fv region of immunoglobulin MCPC 603, for which walk-through mutagenesis was performed on three CDR regions, including CDR1 (using the predetermined amino acid Asp), CDR2 (using the predetermined amino acid His), and CDR3 (using the predetermined amino acid Ser) of the heavy (H) chain.

FIG.2 illustrates the design of "degenerate" oligonucleotides for CDR1.

FIG.3 illustrates the design of "degenerate" oligonucleotides for CDR2.

FIG.4 illustrates the design of "degenerate" oligonucleotides for CDR3.

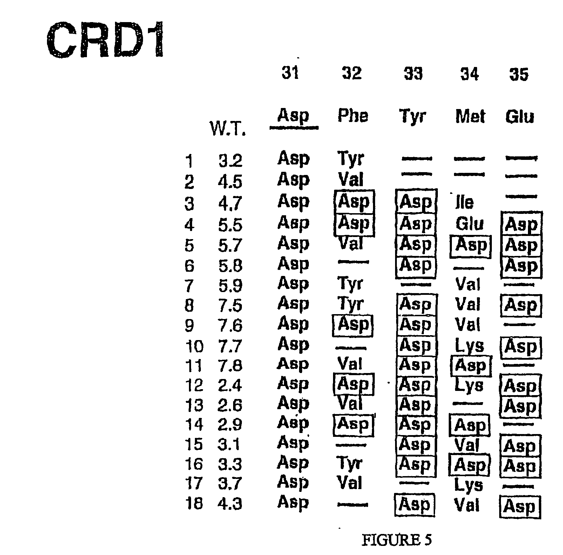

FIG. 5 illustrates the amino acid sequences of the target region, resulting from walk-through mutagenesis in the CDR1 region.

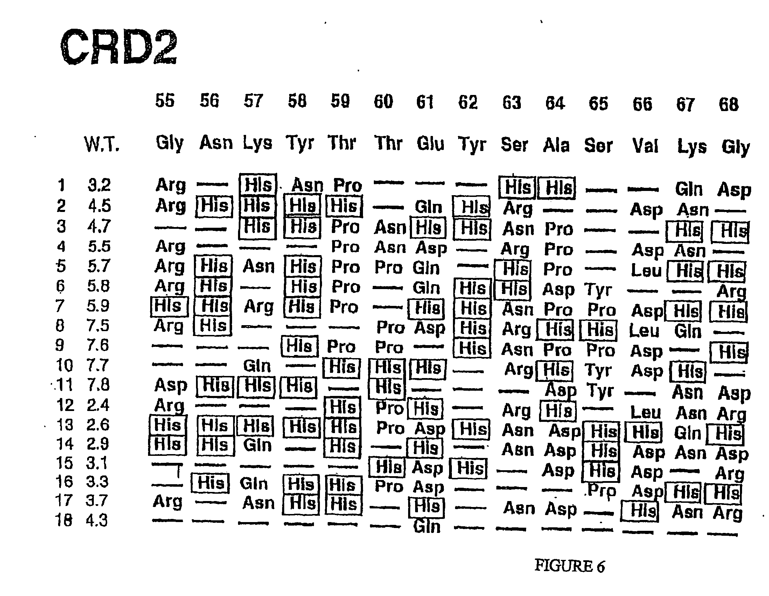

FIG. 6 illustrates the amino acid sequences of the target region, resulting from walk-through mutagenesis in the CDR2 region.

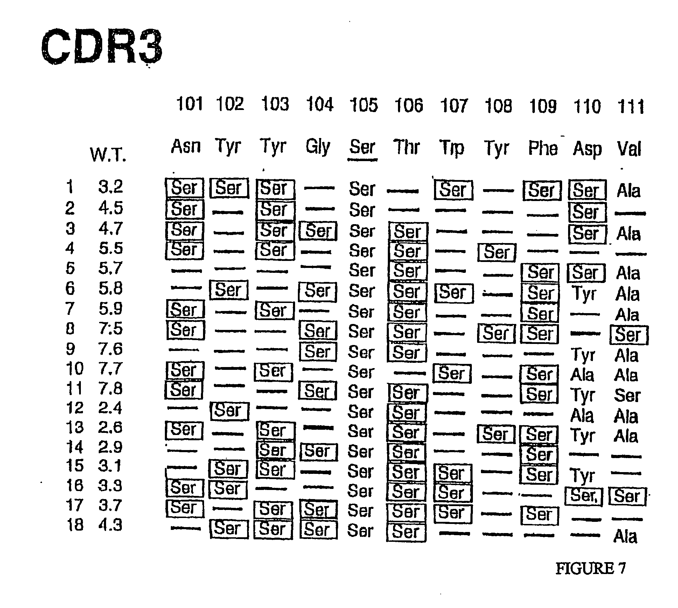

FIG. 7 illustrates the amino acid sequences of the target region, resulting from walk-through mutagenesis in the CDR3 region.

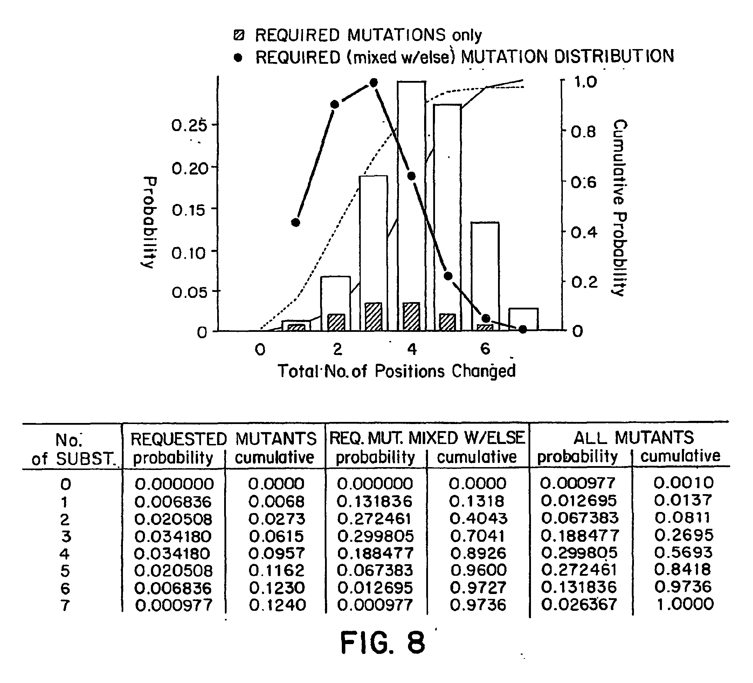

FIG. 8 is a graphic representation of the distribution of mutants, in which a 1:1 ratio of wild-type (prototype):mutant (non-wild-type) nucleic acids were employed during walk-through mutagenesis.

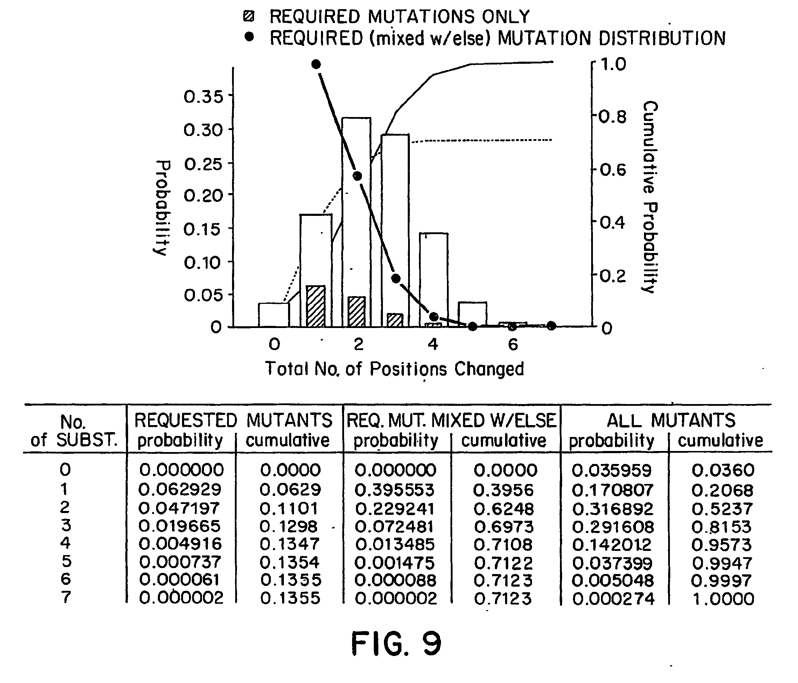

FIG. 9 is a graphic representation of the distribution of mutants, in which a 4:1 ratio of wild-type (prototype):mutant (non-wild-type) nucleic acids were employed during walk-through mutagenesis.

FIG. 10 is a graphic representation of the distribution of mutants, in which a 9:1 ratio of wild-type (prototype):mutant (non-wild-type) nucleic acids were employed during walk-through mutagenesis.

FIG. 11 illustrates the amino acid sequences of the target region (CDR2) of a set of polypeptides prepared by walk-through mutagenesis, in which a 9:1 ratio of wild-type (prototype):mutant (non-wild-type) nucleic acids were employed during walk-through mutagenesis.

FIG. 12 is a graphic representation of a binomial distribution for which the probability of success *p* is 0.2.

FIG. 13 is a graphic representation of a binomial distribution for which the probability of success *p* is 0.1.

DETAILED DESCRIPTION OF THE INVENTION

The present invention relates to methods of walk-through mutagenesis in which "doping" is used to alter the ratio of concentrations of the polypeptide products. In walk-through mutagenesis, libraries of nucleic acids encoding variants of a polypeptide (mutated polypeptides) are produced in which wild-type nucleotides forming codons for an amino acid within a target region, are replaced with non-wild-type nucleotide(s), yielding a mixture of synthetic oligonucleotides designed to produce predictable codon variations. Expression of the library of nucleic acids yields a set of polypeptides in which a predetermined amino acid is introduced in each and every position of a target region of the polypeptide, Doping allows production of mixtures of specific oligonucleotides in particular ratios, in order to produce desired combinations of polypeptide products.

*"Walk-through Mutagenesis"*

"Walk-through mutagenesis" is described in detail in U.S. Patents 5,830,650 and 5,798,208. Walk-through mutagenesis is equally applicable to a wide variety of proteins and polypeptides, including enzymes, immunoglobulins, hormones, cytokines, integrins, and other proteins or polypeptides. To facilitate discussion, the term "polypeptide" is used herein.

One or more "target" regions are selected for the polypeptide. The "target" region(s) can be one or more active regions of the polypeptide, such as a binding site of an enzyme or a hypervariable loop (CDRs) of an immunoglobulin; alternatively, the entire polypeptide can be the "target" region. Regions of the polypeptide that are not subjected to mutagenesis (i.e., those outside the "target" region, if any are outside the target region) are referred to herein as the "constant" region(s). Importantly, several different "target" regions can be mutagenized simultaneously. The same or a different predetermined amino acid can be "walked-through" each target region. This enables the evaluation of amino acid substitutions in conformationally related regions such as the regions which, upon folding of the polypeptide, are associated to make up a functional site such as the catalytic site of an enzyme or the binding site of an antibody.

In walk-through mutagenesis, a set (library) of polypeptides is generated in which a single predetermined amino acid is incorporated at least once into each position of the target region(s) of interest in the polypeptide. The polypeptides resulting from such mutagenesis (referred to herein as "mutated polypeptides") differ from the prototype polypeptide, in that they have the single predetermined amino acid incorporated into one or more positions within one or more target regions of the polypeptide, in lieu of the "wild-type" or "prototype" amino acid which was present at the same position or positions in the prototype polypeptide. The set of mutated polypeptides includes individual mutated polypeptides for each position of the target region(s) of interest; thus, for each position in the target region of interest (e.g., a binding site or CDR) the mixture of mutated polypeptides contains polypeptides that have either an amino acid found in the prototype polypeptide, or the predetermined amino acid, and the mixture of all mutated polypeptides contains all possible variants. The mixture of mutated polypeptides may also contain polypeptides that have neither the predetermined amino acid, nor the prototype amino acid; as discussed below, if the codon encoding the predetermined amino acid requires alteration of more than one nucleotide in order to form the codon that encodes the predetermined amino acid, certain polypeptides may contain amino acids that are encoded by a codon formed by inclusion of less than all the changes necessary to yield the predetermined amino acid. The proportions of each polypeptide depend on the ratios of the concentrations of the nucleotides available during synthesis, as described in detail below.

In walk-through mutagenesis, a predetermined amino acid is selected for the targeted region. If the polypeptide contains more than one targeted region, the same predetermined amino acid can be used for each region; alternatively, different predetermined amino acids can be used for each region. The predetermined amino acid can be a naturally occurring amino acid. The twenty naturally occurring amino acids differ only with respect to their side chain. Each side chain is responsible for chemical properties that make each amino acid unique (see, e.g., Principles of Protein Structure, 1988, by G. E. Schulz and R. M. Schirner, Springer-Verlag). Typical polar and neutral side chains are those of Cys, Ser, Thr, Asn, Gln and Tyr. Gly is also considered to be a borderline member of this group. Ser and Thr play an important role in forming hydrogen-bonds. Thr has an additional asymmetry at the beta carbon, therefore only one of the stereoisomers is used. The acid amide Gln and Asn can also form hydrogen bonds, the amido groups functioning as hydrogen donors and the carbonyl groups functioning as acceptors. Gln has one more CH2 group than Asn, which renders the polar group more flexible and reduces its interaction with the main chain. Tyr has a very polar hydroxyl group (phenolic OH) that can dissociate at high pH values. Tyr behaves somewhat like a charged side chain; its hydrogen bonds are rather strong.

Neutral polar acids are found at the surface as well as inside protein molecules. As internal residues, they usually form hydrogen bonds with each other or with the polypeptide backbone. Cys can form disulfide bridges. Histidine (His) has a heterocyclic aromatic side chain with a pK value of 6.0. In the physiological pH range, its imidazole ring can be either uncharged or charged, after taking up a hydrogen ion from the solution. Since these two states are readily available, His is quite helpful in catalyzing chemical reactions, and is found in the active centers of many enzymes.

Asp and Glu are negatively charged at physiological pH. Because of their short side chain, the carboxyl group of Asp is rather rigid with respect to the main chain; this may explain why the carboxyl group in many catalytic sites is provided by Asp rather than by Glu. Charged acids are generally found at the surface of a protein.

Lys and Arg are frequently found at the surface. They have long and flexible side chains. Wobbling in the surrounding solution, they increase the solubility of the protein globule. In several cases, Lys and Arg take part in forming internal salt bridges or they help in catalysis. Because of their exposure at the surface of the proteins, Lys is a residue more frequently attacked by enzymes which either modify the side chain or cleave the peptide chain at the carbonyl end of Lys residues.

In a preferred embodiment, the predetermined amino acid is one of the following group of amino acids: Ser, Thr, Asn, Gln, Tyr, Cys, His, Glu, Asp, Lys, and Arg. However, any of the twenty naturally occurring amino acids can be selected.

During walk-through mutagenesis, a mixture of oligonucleotides (e.g., cDNA) is prepared, the oligonucleotides encoding all or a portion (the "target region(s)") of the polypeptide of interest. Mutated polypeptides can then be prepared using the mixture of oligonucleotides. In one embodiment, a nucleic acid encoding a mutated polypeptide can be prepared by joining together nucleotide sequences encoding regions of the polypeptide that are not targeted by walk-through mutagenesis (e.g., constant regions), with nucleotide sequences encoding regions of the polypeptide that are targeted by the walk-through mutagenesis. For example, in one embodiment, a nucleic acid encoding a mutated polypeptide can be prepared by joining together nucleotide sequences encoding the constant regions of the polypeptide, with nucleotide sequences encoding the target region(s). Alternatively, nucleotide sequences encoding the target region(s) (e.g., oligonucleotides which are subj ected to incorporation of nucleotides that encode the predetermined amino acid) can be individually inserted into a nucleic acid encoding the prototype polypeptide, in place of the nucleotide sequence encoding the amino acid sequence of the target region(s). If desired, the nucleotide sequences encoding the target region(s) can be made to contain flanking recognition sites for restriction enzymes (see, e.g., U.S. Pat. No. 4,888,286), or naturally-occurring restriction enzyme recognition sites can be used. The mixture of oligonucleotides can be introduced subsequently by cloning them into an appropriate position using the restriction enzyme sites.

For example, a mixture of oligonucleotides can be prepared, in which each oligonucleotide either contains nucleotides encoding the wild-type target region of the prototype polypeptide (or a portion of a target of the prototype polypeptide),or contains one or more nucleotides forming a codon encoding the predetermined amino acid in lieu of one or more native amino acids in the target region. The mixture of oligonucleotides can be produced in a single synthesis by incorporating, at each position within the oligonucleotide, either a nucleotide required for synthesis of the amino acid present in the prototype polypeptide (herein referred to as a "prototype nucleotide") or (in lieu of that nucleotide) a single appropriate nucleotide required for a codon of the predetermined amino acid (a "predetermined nucleotide"). The synthesis of the mixture of oligonucleotides can be performed using an automated DNA synthesizer programmed to deliver either the prototype nucleotide, or the predetermined nucleotide, or a mixture of the two nucleotides, in order to generate an oligonucleotide mixture comprising not only oligonucleotides that encode the target region of the prototype polypeptide, but also oligonucleotides that encode the target region of a mutant polypeptide.

For example, a total of 10 reagent vessels, four of which containing the individual bases and the remaining 6 containing all of the possible two base mixtures among the 4 bases, can be employed to synthesize any mixture of oligonucleotides for the walk-through mutagenesis process. For example, the DNA synthesizer can be designed to contain the following ten chambers:

Table 1: Synthons for Automated DNA Synthesis

| Chamber | Synthon |
|---------|---------|
| 1 | A |
| 2 | T |
| 3 | C |
| 4 | G |
| 5 | (A + T) |
| 6 | (A + C) |
| 7 | (A + G) |
| 8 | (T + C) |
| 9 | (T + G) |
| 10 | (C + G) |

With this arrangement, any nucleotide can be replaced by either one of a combination of two nucleotides at any position of the sequence. Alternatively, if mixing of individual bases in the lines of the oligonucleotide synthesizer is possible, the machine can be programmed to draw from two or more reservoirs of pure bases to generate the desired proportion of nucleotides.

*"Doping" in Walk-through Mutagenesis*

In previously described methods of walk-through mutagenesis (U.S. Patents 5,830,650 and 5,798,208), the two nucleotides (i.e., the wild-type (prototype) nucleotide, and the non-wild-type (predetermined) nucleotide) were used in approximately equal concentrations for the reaction so that there would be an equal chance of incorporating either one into the sequence at the position. Assuming a 50/50 ratio of wild-type and non-wild-type nucleotides, if only one nucleic acid base change is required to mutate a wild-type codon into the codon encoding the predetermined amino acid, one would expect that half (50%) of the nucleic acid sequences produced would contain the codon encoding the predetermined amino acid, and half (50%) would contain the codon encoding the wild-type amino acid. Similarly, if the number of nucleic acid base changes required to produce the codon encoding the predetermined amino acid is two, one would expect that 25% of the nucleic acid sequences produced would contain the codon encoding the wild-type amino acid; 25% of the nucleic acid sequences produced would contain the codon encoding the predetermined amino acid; and 50% (2 X 25%) would contain a codon encoding additional amino acids encoded by the combinatorial nucleotide arrangement.

In the present invention, the ratio of the concentrations of the two nucleotides that are available during synthesis is altered to increase the likelihood that one or the other will be incorporated into the oligonucleotide. The ratio is greater than 1:1. Representative embodiments include a ratio greater than 1:1; a ratio equal to or greater than 4:1; a ratio equal to or greater than 7:1; and a ratio equal to or greater than 9:1. An "available" nucleotide is a nucleotide that is present during synthesis so that it can be incorporated into the oligonucleotide during synthesis of the oligonucleotide; for example, a nucleotide that is drawn from a reservoir of an automated oligonucleotide synthesizer, during synthesis of the oligonucleotide(s), is "available". The ratio of available prototype nucleotide to available mutant nucleotide is established so that greater than 50% of the nucleotides and less than 100% are the prototype nucleotides. Preferably, the ratio is established so that the percentage of prototype nucleotides is equal to or greater than 60%, even more preferably equal to or greater than 70%, and even more preferably equal to or greater than 80%. In particularly preferred embodiments, the ratio is established so that the percentage of prototype nucleotides is equal to or greater than 90%, equal to or greater than 95%, or equal to 99%. For example, the ratio of 9:1, prototype:mutant, (i.e., 90% prototype) will yield a library that contains primarily zero, one or two targeted amino acid substitutions per target region. In one embodiment, the ratio is determined using a binomial distribution that takes into consideration the length of the target region and a desired degree of success in incorporating nucleotides that encode the predetermined amino acid, as described below in relation to the mathematical analysis of doping.

*Mathematical Analysis of Doping*

For a prototype polypeptide of length N to be mutagenized using walk-through mutagenesis, under a probabilistic point of view, the mutagenesis of the polypeptide (with the entire polypeptide as the target region) can be seen as a set of N independent mutagenesis events, one for each amino acid position. It is assumed that there are two possible outcomes at each position: "successful," indicating that in that position, the predetermined amino acid has been introduced; and "unsuccessful," indicating either the wild-type amino acid remains, or an alternate ("undesired") amino acid, which is neither the predetermined amino acid nor the wild-type amino acid, has been introduced. An "undesired" amino acid occurs, for example, when 2 or 3 base mutations in a codon are introduced. The probability of a successful outcome is referred to with the notation *p(j)*, where *j* is the position in the sequence *(1 ≤ j ≤ N)*. The probability of an unsuccessful outcome in the same position *j* is *1-p(j)*. The use of parentheses (in place of the more common subscript) emphasizes the dependence of this probability on the position *j*. In fact, *p(j)* is a function of position *j*, being a function of the base-mix required to obtain the predetermined amino acid (1, 2 or 3 nucleotide base substitutions).

Let X be the discrete random variable representing the total number of mutated amino acids in a sequence of length *N*, whose sample space is:

$$\Omega = \{0,1,2,3...N\}$$

Thus, the following sets can be defined:

$$S_k = \{j \in (1,2,3...N) \vee positions\ of\ success \}$$

$$S'_{N-k} = \{j \in (1,2,3...N) \vee positions\ of\ not\ success \}$$

$$S_k \cap S'_{N-k} = \varnothing$$

Note that the subset indices refer to the cardinality of each set. In the most general situation the equation describing this kind of distribution is:

$$P_k^N = \sum_{i=1}^{\binom{N}{k}} (\prod_{j \in S_i} p(j))( \prod_{j \in S'_{N-k}} (1-p(j))) \qquad 1 \le k \le N$$

which represents the probability to have *k* successes (i.e. predetermined amino acids) out of N independent events.

The number of variants introduced on each experiment should also be considered. The standard walk-through mutagenesis (WTM) (i.e., without doping) was run with a fixed base-mix ratio of 50:50. Under this situation, *p(j)* can assume 3 possible values, depending on the distance *d* between the predetermined amino acid and the wild-type amino acid in position *j*, wherein distance *d* is the number of base mutations required to change the wild-type codon to the predetermined codon (codon encoding the predetermined amino acid):

| Distance | p(j) | Number of variants/position after WTM |
|---|---|---|
| d=1 | p(j)=0.5 | 1 WT + 1 TARGET + 0 EXTRAS = 2 TOTAL |
| d=2 | p(j)=0.25 | 1 WT + 1 TARGET + 2 EXTRAS = 4 TOTAL |
| d=3 | p(j)=0.125 | 1 WT + 1 TARGET + 6 EXTRAS = 8 TOTAL |

Under this hypothesis, standard WTM (without doping) of a single polypeptide of interest is expected to yield a library containing $n$ mutated polypeptides (also referred to as " variants"), for which $n = 2^M$, where $M$ is the total number of predetermined nucleotide bases. The probability to have each variant is $1/n =constant$, independent of the type of amino acid mutations in that sequence. This is because the predetermined nucleotides introduced in each codon can produce, independently of the distance as seen above, only three different sets of 2, 4 or 8 amino acids, with a constant probability of occurrence (50%, 25%, 12.5% respectively). For this reason, the probability of finding a mutated polypeptide (variant) with all the N mutated (predetermined) amino acids in a given library produced by WTM, is exactly the same as finding another with only one mutated (predetermined) amino acid in the same library produced by WTM. This is not desirable for several reasons.

First, in nature it is very improbable (if not impossible) to find a polypeptide with a target sequence (even if short), that after evolution has substitution of most or all of its residues substituted with the same (predetermined) amino acid. Second, the number of variants increase with en exponential law of the type $2^M$, where $M$ is the total number of mutated bases (predetermined nucleotides), and in general $M$ increases with the length of the sequence. Moreover, if the target is to mutagenize in the same time several different target regions within a polypeptide of interest (e.g., all the 6 CDRs of an antibody), it is very common to obtain libraries with a very high number of variants. In these situations, it is very helpful to handle a smaller number of variants by limiting the variants produced to only certain desirable ones.

Doping allows production of libraries with a smaller number of mutated amino acids (that is, mutant polypeptides with a smaller number of predetermined amino acids incorporated therein). Doping is achieved at nucleotide level using different base-mix ratio, and keeping the base ratio constant along the sequence where substitutions are required. This means that every time a 2-base mix is necessary in a codon to incorporate predetermined nucleotides to encode the predetermined amino acid, a base-mix ratio that favors the presence of wild type amino acids (by incorporating prototype nucleotides that encode amino acids in the prototype polypeptide) is utilized instead of a ratio that favors the presence of the predetermined amino acids (by incorporating predetermined nucleotides that encode the predetermined amino acid).

Using this approach, the probability $p(j)$ to have a predetermined amino acid in position $j$ (success) is dependent on the distance between wild type and target. For this reason, the three different situations (d=1,2 or 3) are considered, tuning the values to filter sequences with high number of variants. For example, the information below supposes the use of a base-mix ratio of wild-type (WT) to predetermined (target, TGT) of WT:TGT=9:1.

| Distance | p(j) | Number of variants/position after WTM |
|---|---|---|
| d=1 | p(j)=0.1 | 1 WT (90%) + 1 TARGET (10%) + 0 EXTRAS = 2 TOTAL |
| d=2 | p(j)=0.01 | 1 WT (81%) + 1 TARGET (1%) + 2 EXTRAS (18%) = 4 TOTAL |
| d=3 | p(j)=0.001 | 1 WT (72.9%) + 1 TARGET (0.1%) + 6 EXTRAS (27%) = 8 TOTAL |

In this situation, each substituted amino acid still has a probability of occurrence which is dependent on the number of base mutations required to introduce it in the sequence. However, with doping, the variants in the library do not have the same probability of outcome.

Using constant base-mix ratios during the mutagenesis keeps constant the probability of occurrence of wild-type and predetermined bases. If the probability of occurrence of each amino acid substitution is kept so that each variant's occurrence depends only on the number of substitutions in the sequence, then the desired probability of each occurrence for each substitution can be fixed, and the mutagenesis can be set up to use different base mixes ratios depending on the distance between the predetermined amino acid and the wild-type amino acid. In this way each variant's occurrence will be dependent only on the number of substitutions introduced.

For example, assuming now $p(j)=p=const$, the equation presented above takes a format called standard binomial distribution, characterized by the length of the target sequence and the desired probability of success. The standard equation of a binomial distribution is:

$$P(X=k)=\binom{N}{k}p^{k}(1-p)^{n-k} \qquad 1 \leq k \leq N$$

wherein the parameters $n$ and $p$ are, respectively, the length of the target sequence and the desired probability of success for each single event.

Varying $k$ from 0 to $n$, the typical distribution is obtained, where the average and the variance are:

$$\underline{X}= np$$

$$E_x^2= np\ (1\text{-}p)$$

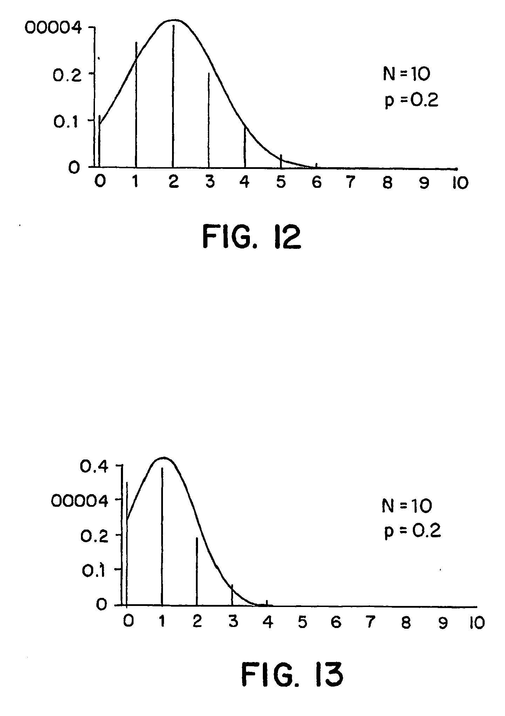

These distributions are depicted in FIG. 12 (p=0.2) and FIG. 13 (p= 0.1).

Once the value of parameters is fixed, the base-mix ratios can be altered to obtain the desired value of p. Different base-mix ratios can be used according to the distance $d$ between wild-type and predetermined amino acids. For example:

$p=0.25$ $n=10$ $\underline{X}=2.5$ $Var(X)=1.87$

| Distance | Ratio (WT:TGT) | Theoric p | Real p |
|---|---|---|---|
| d=1 | 75:25 | 75:25 | 75:25 |
| d=2 | 50:50 | 75:25 | 75:25 |
| d=3 | 37:63 | 75:25 | 75:25 |

$P=0.2$ $n=10$ $\underline{X}=2.0$ $Var(X)=1.6$

| Distance | Ratio (WT: TGT) | Theoric p | Real p |
|---|---|---|---|
| d=1 | 80:20 | 80:20 | 80:20 |
| d=2 | 55:45 | 80:20 | 79.75:20.25 |
| d=3 | 40:60 | 80:20 | 78.5:21.5 |

$P=0.3$ $n=10$ $\underline{X}= 3.0$ $Var(X)=2.1$

| Distance | Ratio (WT:TGT) | Theoric p | Real p |
|---|---|---|---|
| d=1 | 70:30 | 70:30 | 70:30 |
| d=2 | 45:55 | 70:30 | 69.75:30.25 |
| d=3 | 33:67 | 70:30 | 70:30 |

Thus, using these formulae, the desired level of mutated polypeptides for each walk-through mutagenesis can be determined, and the ratios of prototype nucleotides and mutant nucleotides for doping during the walk-through mutagenesis can be adjusted accordingly.

*Preparation of Libraries*

A nucleic acid library containing nucleic acids encoding prototype and mutant polypeptides can then be prepared from such oligonucleotides, as described above, and a polypeptide library containing the prototype and mutant polypeptides themselves can then be generated from the nucleic acids, using standard techniques. For example, the nucleic acids encoding the mutated immunoglobulins can be introduced into a host cell for expression (see, e.g., Huse, W. D. et al., Science 246: 1275 (1989); Viera, J. et al., Meth. Enzymol. 153: 3 (1987)). The nucleic acids can be expressed, for example, in an *E. coli* expression system (see, e.g., Pluckthun, A. and Skerra, A., Meth. Enzymol. 178:476-515 (1989) ; Skerra, A. et al., Biotechnology 9:23-278 (1991)). They can be expressed for secretion in the medium and/or in the cytoplasm of bacteria (see, e.g., Better, M. and Horwitz, A., Meth. Enzymol. 178:476 (1989)); alternatively, they can be expressed in other organisms such as yeast or mammalian cells (e.g., myeloma or hybridoma cells).

One of ordinary skill in the art will understand that numerous expression methods can be employed to produce libraries described herein. By fusing the nucleic acids to additional genetic elements, such as promoters, terminators, and other suitable sequences that facilitate transcription and translation, expression in vitro (ribosome display) can be achieved as described by Pluckthun et al.(Pluckthun, A. and Skerra, A., Meth. Enzymol. 178:476-515 (1989)). Similarly, Phage display, bacterial expression, baculovirus-infected insect cells, fungi (yeast), plant and mammalian cell expression can be obtained as described (Antibody Engineering. R. Konterman, S.Dubel (Eds.). Springer Lab manual. Spriger-Verlag. Berlin, Heidelberg (2001), Chapter 1, "Recombinant Antibodies by S. Dubel and R. E. Konterman. Pp. 4-16). Libraries of scFV can also be fused to other genes to produce chimaeric proteins with binding moieties (Fv) and other functions, such as catalytic, cytotoxic, etc. (Antibody Engineering. R. Konterman, S. Dubel (Eds.). Springer Lab manual. Spriger-Verlag. Berlin, Heidelberg (2001), Chapter 41. Stabilization Strategies and Application of recombinant Fvs and Fv Fusion proteins. By U. Brinkmann, pp. 593-615).

The methods of the invention allow production of polypeptide mutants in which the overall presence (walk through) of the predetermined amino acid is limited to one or two positions per mutated polypeptide, leaving the remaining amino acids in the targeted region intact or as close as possible to the prototype sequence. In this way, more precise and specific chemical variations can be produced. For example, in order to achieve binding improvement between two proteins, or between an antibody and an antigen, one may explore the systematic effect of the presence of an additional hydrophobic side chain across the binding regions (as the "target" regions), position by position. Similarly, by selecting for the predetermined amino acid, an amino acid with specific chemical properties, one can address the effect of charge (+ or -), lipophylicity, hydrophylicity, etc., on the overall binding process.

*Immunoglobulins*

In one particular embodiment, the polypeptide of interest is an immunoglobulin. As used herein, the term "immunoglobulin" can refer to a full-length immunoglobulin, as well as to a portion thereof that contains the variable regions (e.g., an Fab fragment) of an immunoglobulin. The immunoglobulin that is the polypeptide of interest can be from any species that generates antibodies, preferably a mammal, and particularly a human; alternatively, the immunoglobulin of interest can be a chimeric antibody or a "consensus" or canonic structure generated from amino acid data banks for antibodies (Kabat et al. ((1991) Sequences of proteins of Immunological Interest. 5th Edition. US Department Of Health and Human Services, Public Service, NIH.)). The immunoglobulin of interest can be a wild-type immunoglobulin (e.g., one that is isolated or can be isolated from an organism, such as an immunoglobulin that can be found in an appropriate physiological sample (e.g., blood, serum, etc.) from a mammal, particularly a human). Alternatively, the immunoglobulin of interest can be a modified immunoglobulin (e.g., an previously wild-type immunoglobulin, into which alterations have been introduced into one or more variable regions and/or constant regions).

In one embodiment of the invention, the immunoglobulin of interest is a catalytic antibody. An immunoglobulin can be made catalytic, or the catalytic activity can be enhanced, by the introduction of suitable amino acids into the binding site of the immunoglobulin's variable region (Fv region) in the methods described herein. For instance, catalytic triads modeled after serine proteases can be created in the hypervariable segments of the Fv region of an antibody and screened for proteolytic activity. Representative catalytic antibodies include oxidoreductases, transferases, hydrolases, lyases, isomerases and ligases; these categories include proteases, carbohydrases, lipases, dioxygenases and peroxidases, as well as other enzymes. These and other enzymes can be used for enzymatic conversions in health care, cosmetics, foods, brewing, detergents, environment (e.g., wastewater treatment), agriculture, tanning, textiles, and other chemical processes, such as diagnostic and therapeutic applications, conversions of fats, carbohydrates and protein, degradation of organic pollutants and synthesis of chemicals. For example, therapeutically effective proteases with fibrinolytic activity, or activity against viral structures necessary for infectivity, such as viral coat proteins, could be engineered. Such proteases could be useful anti-thrombotic agents or anti-viral agents against viruses such as AIDS, rhinoviruses, influenza, or hepatitis. Alternatively, in another example, oxygenases (e.g., dioxygenases), a class of enzymes requiring a co-factor for oxidation of aromatic rings and other double bonds, have industrial applications in biopulping processes, conversion of biomass into fuels or other chemicals, conversion of waste water contaminants, bioprocessing of coal, and detoxification of hazardous organic compounds.

The methods of the invention may be particularly useful in generation of universal libraries for immunoglobulins, as discussed in greater detail in U.S. Patent application Serial No. 60/373,558.

*Library Uses*

Libraries as described herein encode, or contain, mutated polypeptides which have been generated in a manner that allows systematic and thorough analysis of the binding regions of the prototype polypeptide, and particularly, of the influence of a particular preselected amino acid on the binding regions. The libraries avoid problems relating to control or prediction of the nature of a mutation associated with random mutagenesis; allow generation of specific information on the very particular mutations that allow altered interaction of the polypeptide of interest with other agents (e.g., ligands, receptors, antigens), including multiple interactions by amino acids in the varying binding regions of the polypeptide of interest.

The libraries can be screened by appropriate means for particular polypeptides, such as immunoglobulins having specific characteristics. For example, catalytic activity can be ascertained by suitable assays for substrate conversion and binding activity can be evaluated by standard immunoassay and/or affinity chromatography. Assays for these activities can be designed in which a cell requires the desired activity for growth. For example, in screening for immunoglobulins that have a particular activity, such as the ability to degrade toxic compounds, the incorporation of lethal levels of the toxic compound into nutrient plates would permit the growth only of cells expressing an activity which degrades the toxic compound (Wasserfallen, A., Rekik, M., and Harayama, S., Biotechnology 9: 296-298 (1991)). Libraries can also be screened for other activities, such as for an ability to target or destroy pathogens. Assays for these activities can be designed in which the pathogen of interest is exposed to the antibody, and antibodies demonstrating the desired property (e.g., killing of the pathogen) can be selected.

The following Exemplification is offered for the purpose of illustrating the present invention and are not to be construed to limit the scope of this invention. The teachings of all references cited are hereby incorporated herein in their entirety.

EXEMPLIFICATION

A. Material and Methods

To assess the effect of doping on walk-through mutagenesis, walk-through mutagenesis was performed on three of the hypervariable regions or complementarity determining regions (CDRs) of the monoclonal antibody MCPC 603. MCPC 603 is a monoclonal antibody that binds phosphorylcholine. This immunoglobulin is recognized as a good model for investigating binding and catalysis because the protein and its binding region have been well characterized structurally. The CDRs for the MCPC 603 antibody have been identified. In the heavy chain, CDR1 spans amino acids 31-35, CDR2 spans 50-69, and CDR3 spans 101-111. In the light chain, the amino acids of CDR1 are 24-40, CDR2 spans amino acids 55-62, and CDR3 spans amino acids 95-103.

CDR1, CDR2 and CDR3 of the heavy chain (VH) were the domains selected. The published amino acid sequence of the MCPC 603 VH and VL regions can be converted to a DNA sequence (Rudikoff, S. and Potter, M., Biochemistry 13: 4033 (1974)); alternatively, the wild type DNA sequence of MCPC 603 can be used (Pluckthun, A. et al., Cold Spring Harbor Symp. Quant. Biol., Vol. LII: 105-112 (1987)). Restriction sites can be incorporated into the sequence to facilitate introduction of degenerate oligonucleotides or the degenerate sequences may be introduced at the stage of gene assembly.

The predetermined amino acids selected for the walk-through mutagenesis were the three residues of the catalytic triad of serine proteases, Asp, His and Ser. Asp was selected for VH CDR1, His was selected for VH CDR2, and Ser was selected for VH CDR3.

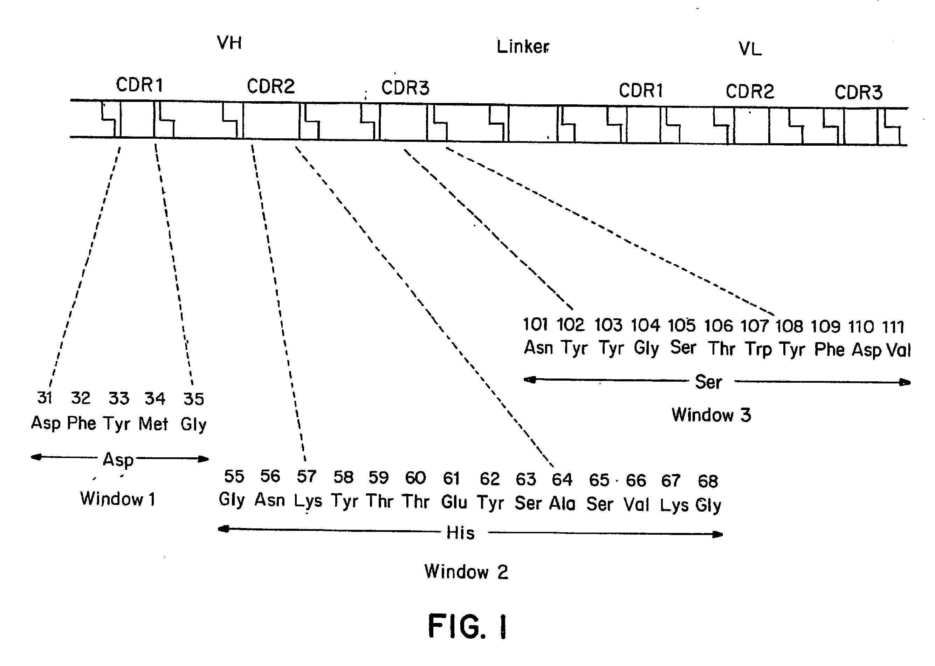

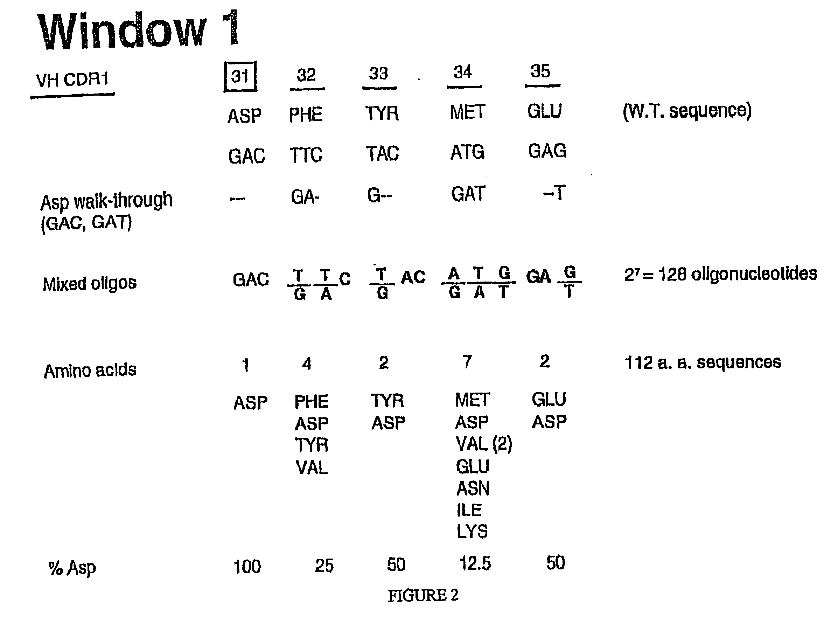

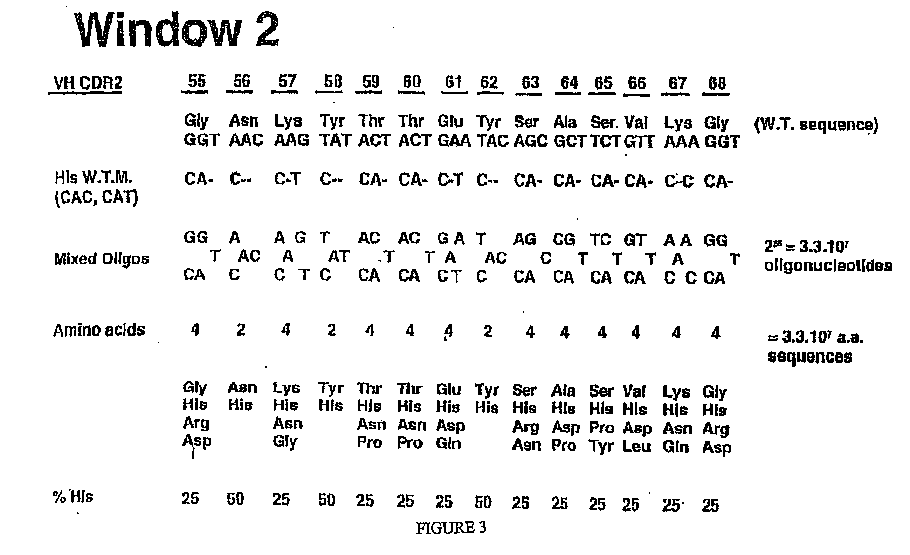

The structure of the gene used for walk-through mutagenesis in the CDRs of MCPC 603 is shown in FIG. 1; the positions or "windows" to be mutagenized are shown. It is understood that the oligonucleotide synthesized can be larger than the window shown to facilitate insertion into the target construct. The mixture of oligonucleotides corresponding to the VH CDR1 is designed in order to substitute each wild-type (prototype) amino acid with Asp (FIG. 3a). Two codons specify asp (GAC and GAT). The first codon of CDR1 does not require any substitution. The second codon (TTC, Phe) requires substitution at the first (T to G) and second position (T to A) in order to convert it into a codon for Asp. The third codon (TAC, Tyr) requires only one substitution at the first position (T to G). The fourth codon (ATG, Met) requires three substitutions, the first being A to G, the second T to A and the third G to T. The fifth codon (GAG, Glu) requires only one substitution at the third position (G to T). The resulting mixture of oligonucleotides is depicted in Figure 2.

From the genetic code, it is possible to deduce all the amino acids that will substitute the original amino acid in each position. For this case, the first amino acid will always be Asp (100%), the second will be Phe (25%), Asp (25%), Tyr (25%) or Val (25%), the third amino acid will be Tyr (50%) or Asp (50%); the fourth will be Met (12.5%), Asp (12.5%), Val (25%), Glu (12.5%), Asn (12.5%), Ile (12.5%) or Lys (12.5%); and the fifth codon will be either Glu (50%) or Asp (50%). In total, 128 oligonucleotides which will code for 112 different protein sequences could be generated. Among the 112 different amino acid sequences generated will be the wild type (prototype) sequence (which has an Asp residue at position 31), and sequences differing from wild type in that they contain from one to four Asp residues at positions 32-35, in all possible permutations (see FIG. 2). In addition, some sequences, either with or without Asp substitutions, will contain an amino acid-neither wild type nor Asp- at positions 32, 34 or both. These amino acids are introduced by permutations of the nucleotides which encode the wild type amino acid and the preselected amino acid. For example, in FIG. 2, at position 32, tyrosine (Tyr) and valine (Val) are generated in addition to the wild type phenylalanine (Phe) residue and the preselected Asp residue.

The CDR2 of the VH region of MCPC603 contains 14 amino acids (55-68), as shown in FIG. 3. The mixture of oligonucleotides is designed in which each amino acid of the wild type sequence will be replaced by histidine (His). Two codons (CAT and CAC) specify His. The substitutions required throughout the wild-type DNA sequence total 25. Thus, the oligonucleotide mixture produced contains oligonucleotides which specify $3.3 \times 10^7$ different peptide sequences (see FIG. 3).

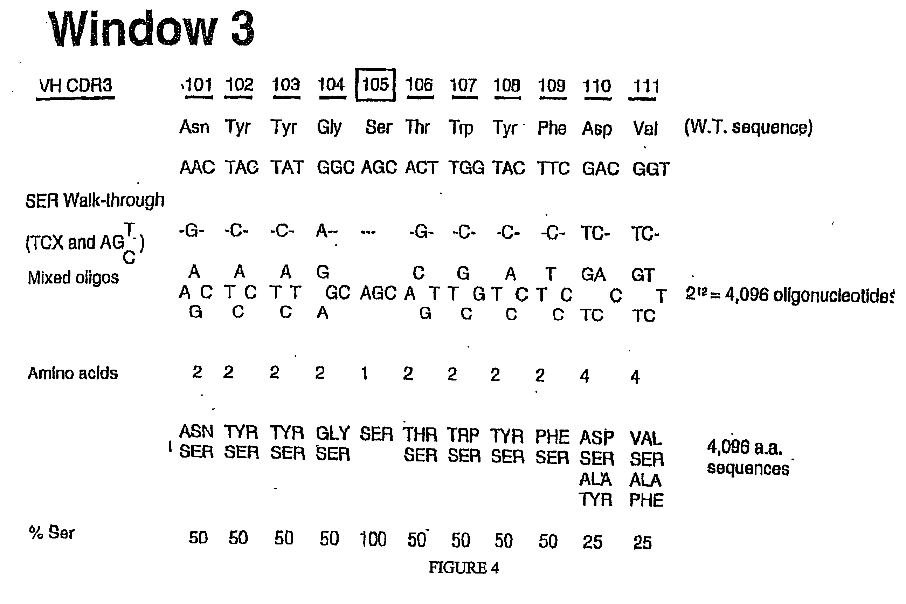

The CDR3 of the VH region of MCPC603 is made up of 11 amino acids, as shown in FIG. 4. A mixture of oligonucleotides is designed in which each non-serine amino acid of the wild type sequence is replaced by serine (Ser), as described above for CDR1. Six codons (TCX and AGC, AGT) specify Ser. The substitutions required throughout the wild-type sequence amount to 12. As a result, the oligonucleotide mixture produced contains 4096 different oligonucleotides which, in this case, will code for 4096 protein sequences. Among these sequences will be some containing a single serine residue (in addition to the serine 105) in any one of the other positions (101-104, 106-111), as well as variants with more than one serine, in any combination (see FIG. 4).

Using walk-through mutagenesis, a library of Fv sequences was produced which contains several different protein sequences, including the prototype and the mutants. A significant proportion of these sequences will encode the amino acid triad His, Ser, Asp typical of serine proteases at the desired positions within the targeted hypervariable regions. The walk-through mutagenesis was performed by synthesis of the degenerate mixture of oligonucleotides in an automated DNA synthesizer programmed to deliver either one nucleotide to the reaction chamber or a mixture of two nucleotides in *equal* ratio, mixed prior to the delivery to reaction chamber.

Each mixture of synthetic oligonucleotides was inserted into the gene for the respective MCPC 603 variable region. The oligonucleotides were converted into double-stranded chains by enzymatic techniques (see e.g., Oliphant, A. R. et al., 1986, supra) and then ligated into a restricted plasmid containing the gene coding for the protein to be mutagenized. The restriction sites were either naturally occurring sites or engineered restriction sites.

The mutant MCPC 603 genes constructed by these or other suitable procedures described above were expressed in a convenient *E. coli* expression system, such as that described by Pluckthun and Skerra. (Pluckthun, A. and Skerra, A., Meth. Enzymol. 178: 476-515 (1989); Skerra, A. et al., Biotechnology 9: 273-278 (1991)).

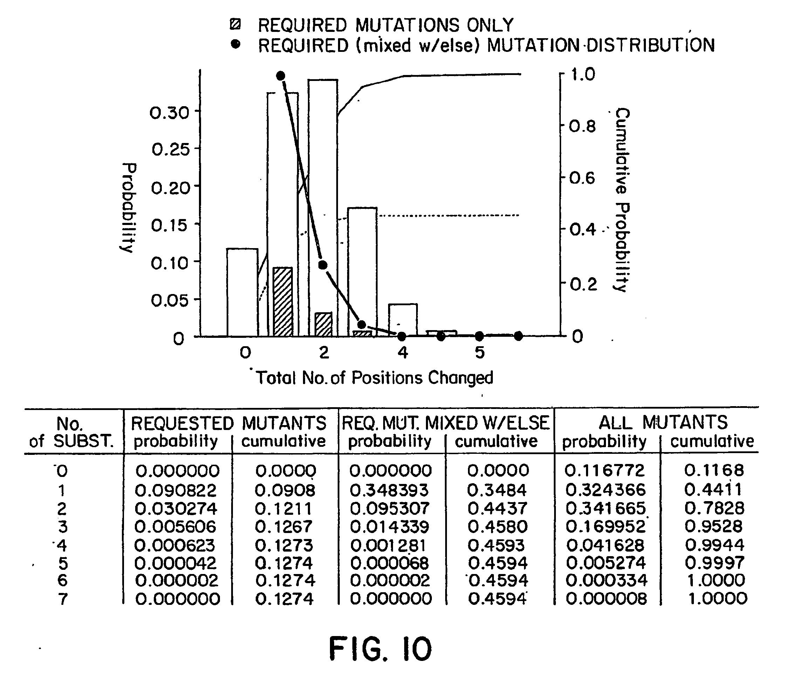

A computer program designed to predict the distribution of mutants was used to assess the effects of "doping" on the ratio of wild-type to mutant bases and the resultant amino acids. The program was used to assess the effects of doping on the VH-CDR2 (Asp) mutant. Results generated using a ratio of 1:1 wild-type (prototype):mutant (non-wild-type) is shown in FIG: 8; results using a ratio of 4:1 are shown in FIG: 9; and results using a ratio of 9:1 1 are shown in FIG. 10. It can be seen that the distribution alters dramatically with the alteration of the ratio.

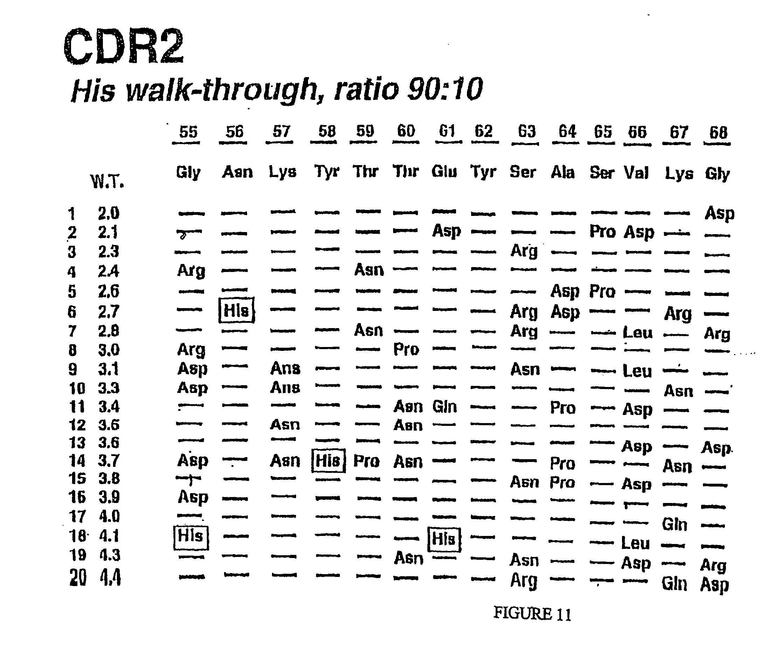

The methods described above were also used to generate a set of mutants of the MOPC603 antibody, using a 9:1 ratio in favor of the wild-type. Twenty new colonies were generated, and sequencing data is shown in FIG. 11. The results confirm that the library contained primarily zero, one or two targeted amino acid substitutions in the target region.

## Claims

1. A method of walk-through mutagenesis of a nucleic acid encoding a prototype polypeptide of interest, comprising:

   a) selecting one or more target region(s) of prototype amino acids in the prototype polypeptide of interest encoded by the nucleic acid;
   b) for each of the target region(s), predetermining one amino acid to be incorporated into the target region in lieu of the prototype amino acids; and
   c) synthesizing a mixture of oligonucleotides comprising a nucleotide sequence for each target region, wherein each oligonucleotide contains, at each sequence position in the target region, either a nucleotide that is required for synthesis of the prototype amino acid of the polypeptide, or a predetermined nucleotide that is required for synthesis of the predetermined amino acid, wherein during synthesis, the ratio of available prototype nucleotides, to available predetermined nucleotides, is equal to or greater than 4:1, wherein said ratio is determined by use of a binominial distribution and wherein said probability distribution takes into consideration the number ($N$) of amino acids in the target region(s) and the probability (P) of success in incorporating nucleotides that encode the predetermined amino acid, and wherein X (total number of mutated amino acids in a sequence of length N) is less than N.

2. The method of claim 1, further comprising generating an expression library of nucleic acids comprising said oligonucleotides.

3. The method of claim 1, wherein the ratio is equal to or greater than:

   a) 7:1; or
   b) 9:1.

4. The method of claim 1, wherein the target region comprises:

   a) a functional domain of the polypeptide;
   b) a catalytic site of an immunoglobulin; or
   c) a hypervariable region of an antibody.

5. The method of claim 1, wherein the predetermined amino acid is Ser, Thr, Asn, Gln, Tyr, Cys, His, Glu, Asp, Lys or Arg.

6. The method of claim 1, wherein the binominial distribution is represented by the equation:

$$P(X=k) = \binom{N}{k} p^k (1-p)^{n-k} \qquad 1 \leq k \leq N$$

**Patentansprüche**

1. Verfahren einer Walk-Through-Mutagenese von einer ein gewünschtes Prototyp-Polypeptid codierenden Nucleinsäure in den Schritten:

   a) Auswahl einer oder mehrerer Zielregion(en) von Prototyp-Aminosäuren in dem von der Nucleinsäure codierten gewünschten Prototyp-Polypeptid;
   b) Vorherbestimmung einer in die Zielregion an Stelle der Prototyp-Aminosäuren einzubauenden Aminosäure für jede (der) Zielregion(en) und
   c) Synthetisieren einer Mischung von Oligonucleotiden mit einer Nucleotidsequenz für jede Zielregion, wobei jedes Oligonucleotid an jeder Sequenzposition in der Zielregion entweder ein Nucleotid enthält, das für die Synthese der Prototyp-Aminosäure des Polypeptids benötigt wird, oder ein vorherbestimmtes Nucleotid enthält, das für die Synthese der vorherbestimmten Aminosäure benötigt wird, wobei das Verhältnis von verfügbaren Prototyp-Nucleotiden zu den verfügbaren vorherbestimmten Nucleotiden während der Synthese gleich oder größer als 4:1 ist, wobei das Verhältnis mit Hilfe einer Binomialverteilung bestimmt wird und diese Wahrscheinlichkeitsverteilung die Anzahl (N) der Aminosäuren in der (den) Zielregion(en) und die Wahrscheinlichkeit (P) für den Erfolg beim Einbau der die vorherbestimmte Aminosäure codierenden Nucleotide berücksichtigt und wobei X (die Gesamtzahl der in einer Sequenz der Länge N mutierten Aminosäuren) kleiner N ist.

2. Verfahren nach Anspruch 1, welches ferner die Erstellung einer Bibliothek von Nucleinsäuren umfasst, welche diese Oligonucleotide enthält.

34

**3.** Verfahren nach Anspruch 1, in welchem das Verhältnis gleich oder größer als

    a) 7:1 oder
    b) 9:1 ist.

**4.** Verfahren nach Anspruch 1, in welchem die Zielregion

    a) eine funktionelle Domäne des Polypeptids,
    b) eine katalytische Stelle eines Immunglobulins und
    c) eine hypervariable Region eines Antikörpers

umfasst.

**5.** Verfahren nach Anspruch 1, in welchem die vorherbestimmte Aminosäure Ser, Thr, Asn, Gln, Tyr, Cys, His, Glu, Asp, Lys oder Arg ist.

**6.** Verfahren nach Anspruch 1, in welchem die Binominalverteilung durch die Gleichung

$$P(X=k)=\binom{N}{k}p^k(1-p)^{n-k} \qquad 1\leq k\leq N$$

ausgedrückt wird.

## Revendications

**1.** Procédé de mutagenèse traversante d'un acide nucléique codant pour un polypeptide prototype d'intérêt, comportant les étapes consistant à:

    a) sélectionner une ou plusieurs régions cibles d'acides aminés prototypes dans le polypeptide prototype d'intérêt codé par l'acide nucléique,
    b) pour chaque région de la ou les régions cibles, déterminer un acide aminé à incorporer dans la région cible à la place des acides aminés prototypes, et
    c) synthétiser un mélange d'oligonucléotides comportant une séquence nucléotidique pour chaque région cible, chaque oligonucléotide contenant, à chaque position de séquence dans la région cible, un nucléotide qui est nécessaire pour la synthèse de l'acide aminé prototype du polypeptide, ou un nucléotide prédéterminé qui est nécessaire pour la synthèse de l'acide aminé prédéterminé, dans lequel, au cours de la synthèse, le rapport entre des nucléotides prototypes disponibles et des nucléotides prédéterminés disponibles, est égal ou supérieur à 4:1, dans lequel ledit rapport est déterminé en utilisant une distribution binomiale et dans lequel ladite distribution de probabilité prend en compte le nombre (N) d'acides aminés dans la ou les régions cibles et la probabilité (P) de succès d'incorporer des nucléotides qui codent pour l'acide aminé prédéterminé, et dans lequel X (nombre total d'acides aminés mutés dans une séquence de longueur N) est inférieure à N.

**2.** Procédé selon la revendication 1, comportant de plus la génération d'une banque d'expression d'acides nucléiques comportant lesdits oligonucléotides.

**3.** Procédé selon la revendication 1, dans lequel le rapport est égal ou supérieur à :

    a) 7:1, ou
    b) 9:1.

**4.** Procédé selon la revendication 1, dans lequel la région cible comporte:

a) un domaine fonctionnel du polypeptide,
b) un site catalytique d'une immunoglobuline, ou
c) une région hypervariable d'un anticorps.

**5.** Procédé selon la revendication 1, dans lequel l'acide aminé prédéterminé est Ser, Thr, Asn, Gln, Tyr, Cys, His, Glu, Asp, Lys ou Arg.

**6.** Procédé selon la revendication 1, dans lequel la distribution binomiale est représentée par l'équation :

$$P(X = k) = \binom{N}{k} p^k (1-p)^{n-k} \qquad 1 \le k \le N$$

FIG. 1

# Window 1

| VH CDR1 | [31] | 32 | 33 | 34 | 35 | |
|---|---|---|---|---|---|---|
| | ASP | PHE | TYR | MET | GLU | (W.T. sequence) |
| | GAC | TTC | TAC | ATG | GAG | |
| Asp walk-through (GAC, GAT) | — | GA- | G-- | GAT | -T | |
| Mixed oligos | GAC | $\frac{T\ T}{G\ A}$C | $\frac{T}{G}$AC | $\frac{A\ T\ G}{G\ A\ T}$ | GA$\frac{G}{T}$ | $2^7 = 128$ oligonucleotides |
| Amino acids | 1 | 4 | 2 | 7 | 2 | 112 a. a. sequences |
| | ASP | PHE ASP TYR VAL | TYR ASP | MET ASP VAL (2) GLU ASN ILE LYS | GLU ASP | |
| % Asp | 100 | 25 | 50 | 12.5 | 50 | |

FIGURE 2

# Window 2

| VH CDR2 | 55 | 56 | 57 | 58 | 59 | 60 | 61 | 62 | 63 | 64 | 65 | 66 | 67 | 68 | |
|---|---|---|---|---|---|---|---|---|---|---|---|---|---|---|---|
| | Gly<br>GGT | Asn<br>AAC | Lys<br>AAG | Tyr<br>TAT | Thr<br>ACT | Thr<br>ACT | Glu<br>GAA | Tyr<br>TAC | Ser<br>AGC | Ala<br>GCT | Ser.<br>TCT | Val<br>GTT | Lys<br>AAA | Gly<br>GGT | (W.T. sequence) |
| His W.T.M.<br>(CAC, CAT) | CA- | C-- | C-T | C-- | CA- | CA- | C-T | C-- | CA- | CA- | CA- | CA- | C-C | CA- | |
| Mixed Oligos | GG<br>T<br>CA | A<br>AC<br>C | A<br>A<br>C | G<br>AT<br>T | T<br>C | AC<br>-T<br>CA | AC<br>T<br>CA | GA<br>A<br>CT | T<br>AC<br>C | AG<br>C<br>CA | CG<br>T<br>CA | TC<br>T<br>CA | GT<br>T<br>CA | AA<br>A<br>C | GG<br>T<br>C CA | $2^{85} = 3.3.10^{t}$<br>oligonucleotides |
| Amino acids | 4 | 2 | 4 | 2 | 4 | 4 | 4 | 2 | 4 | 4 | 4 | 4 | 4 | 4 | $\approx 3.3.10^{7}$ a.a.<br>sequences |
| | Gly<br>His<br>Arg<br>Asp | Asn<br>His | Lys<br>His<br>Asn<br>Gly | Tyr<br>His | Thr<br>His<br>Asn<br>Pro | Thr<br>His<br>Asn<br>Pro | Glu<br>His<br>Asp<br>Gln | Tyr<br>His | Ser<br>His<br>Arg<br>Asn | Ala<br>His<br>Asp<br>Pro | Ser<br>His<br>Pro<br>Tyr | Val<br>His<br>Asp<br>Leu | Lys<br>His<br>Asn<br>Gln | Gly<br>His<br>Arg<br>Asp | |
| % His | 25 | 50 | 25 | 50 | 25 | 25 | 25 | 50 | 25 | 25 | 25 | 25 | 25 | 25 | |

FIGURE 3

EP 1 495 142 B1

# Window 3

| VH CDR3 | 101 | 102 | 103 | 104 | 105 | 106 | 107 | 108 | 109 | 110 | 111 | |
|---|---|---|---|---|---|---|---|---|---|---|---|---|
| | Asn | Tyr | Tyr | Gly | Ser | Thr | Trp | Tyr | Phe | Asp | Val | (W.T. sequence) |
| | AAC | TAC | TAT | GGC | AGC | ACT | TGG | TAC | TTC | GAC | GGT | |

SER Walk-through

(TCX and AG$^{T}_{C}$)

| | 101 | 102 | 103 | 104 | 105 | 106 | 107 | 108 | 109 | 110 | 111 | |
|---|---|---|---|---|---|---|---|---|---|---|---|---|
| | -G- | -C- | -C- | A-- | --- | -G- | -C- | -C- | -C- | TC- | TC- | |
| Mixed oligos | A | A | A | G | | C | G | A | T | GA | GT | |
| | A C | T C | T T | GC | AGC | A T | T G | T C | T C | C | T | $2^{12}=4{,}096$ oligonucleotides |
| | G | C | C | A | | G | C | C | C | TC | TC | |

| Amino acids | 2 | 2 | 2 | 2 | 1 | 2 | 2 | 2 | 2 | 4 | 4 | |
|---|---|---|---|---|---|---|---|---|---|---|---|---|

| | 101 | 102 | 103 | 104 | 105 | 106 | 107 | 108 | 109 | 110 | 111 | |
|---|---|---|---|---|---|---|---|---|---|---|---|---|
| | ASN | TYR | TYR | GLY | SER | THR | TRP | TYR | PHE | ASP | VAL | 4,096 a.a. sequences |
| | SER | SER | SER | SER | | SER | SER | SER | SER | SER | SER | |
| | | | | | | | | | | ALA | ALA | |
| | | | | | | | | | | TYR | PHE | |

| % Ser | 50 | 50 | 50 | 50 | 100 | 50 | 50 | 50 | 50 | 25 | 25 | |
|---|---|---|---|---|---|---|---|---|---|---|---|---|

FIGURE 4

# CRD1

|  | W.T. | 31 Asp | 32 Phe | 33 Tyr | 34 Met | 35 Glu |
|---|---|---|---|---|---|---|
| 1 | 3.2 | Asp | Tyr | — | — | — |
| 2 | 4.5 | Asp | Val | — | — | — |
| 3 | 4.7 | Asp | Asp | Asp | Ile | — |
| 4 | 5.5 | Asp | Asp | Asp | Glu | Asp |
| 5 | 5.7 | Asp | Val | Asp | Asp | Asp |
| 6 | 5.8 | Asp | — | Asp | | Asp |
| 7 | 5.9 | Asp | Tyr | — | Val | |
| 8 | 7.5 | Asp | Tyr | Asp | Val | Asp |
| 9 | 7.6 | Asp | Asp | Asp | Val | — |
| 10 | 7.7 | Asp | — | Asp | Lys | Asp |
| 11 | 7.8 | Asp | Val | Asp | Asp | — |
| 12 | 2.4 | Asp | Asp | Asp | Lys | Asp |
| 13 | 2.6 | Asp | Val | Asp | — | Asp |
| 14 | 2.9 | Asp | Asp | Asp | Asp | |
| 15 | 3.1 | Asp | — | Asp | Val | Asp |
| 16 | 3.3 | Asp | Tyr | Asp | Asp | Asp |
| 17 | 3.7 | Asp | Val | — | Lys | |
| 18 | 4.3 | Asp | — | Asp | Val | Asp |

FIGURE 5

EP 1 495 142 B1

# CRD2

| | | 55 | 56 | 57 | 58 | 59 | 60 | 61 | 62 | 63 | 64 | 65 | 66 | 67 | 68 |
|---|---|---|---|---|---|---|---|---|---|---|---|---|---|---|---|
| | W.T. | Gly | Asn | Lys | Tyr | Thr | Thr | Glu | Tyr | Ser | Ala | Ser | Val | Lys | Gly |
| 1 | 3.2 | Arg | — | His | Asn | Pro | — | — | — | His | His | — | — | Gln | Asp |
| 2 | 4.5 | Arg | His | His | His | His | — | Gln | His | Arg | — | — | Asp | Asn | — |
| 3 | 4.7 | — | — | His | His | Pro | Asn | His | His | Asn | Pro | — | — | His | His |
| 4 | 5.5 | Arg | — | — | — | Pro | Asn | Asp | — | Arg | Pro | — | Asp | Asn | — |
| 5 | 5.7 | Arg | His | Asn | His | Pro | Pro | Gln | — | His | Pro | — | Leu | His | His |
| 6 | 5.8 | Arg | His | — | His | Pro | — | Gln | His | His | Asp | Tyr | — | — | Arg |
| 7 | 5.9 | His | His | Arg | His | Pro | — | His | His | Asn | Pro | Pro | Asp | His | His |
| 8 | 7.5 | Arg | His | — | — | — | Pro | Asp | His | Arg | His | His | Leu | Gln | — |
| 9 | 7.6 | — | — | — | His | Pro | Pro | — | His | Asn | Pro | Pro | Asp | — | His |
| 10 | 7.7 | — | — | Gln | — | His | His | His | — | Arg | His | Tyr | Asp | His | — |
| 11 | 7.8 | Asp | His | His | His | — | His | — | — | — | Asp | Tyr | — | Asn | Asp |
| 12 | 2.4 | Arg | — | — | — | His | Pro | His | — | Arg | His | — | Leu | Asn | Arg |
| 13 | 2.6 | His | His | His | His | His | Pro | Asp | His | Asn | Asp | His | His | Gln | His |
| 14 | 2.9 | His | His | Gln | — | His | — | His | — | Asn | Asp | His | Asp | Asn | Asp |
| 15 | 3.1 | — | — | — | — | — | His | Asp | His | — | Asp | His | Asp | — | Arg |
| 16 | 3.3 | — | His | Gln | His | His | Pro | Asp | — | — | — | Pro | Asp | — | Arg |
| 17 | 3.7 | Arg | — | Asn | His | His | — | His | — | Asn | Asp | — | His | Asn | Arg |
| 18 | 4.3 | — | — | — | — | — | Gln | — | — | — | — | — | — | — | — |

FIGURE 6

EP 1 495 142 B1

# CDR3

| | W.T. | 101 Asn | 102 Tyr | 103 Tyr | 104 Gly | 105 Ser | 106 Thr | 107 Trp | 108 Tyr | 109 Phe | 110 Asp | 111 Val |
|---|---|---|---|---|---|---|---|---|---|---|---|---|
| 1 | 3.2 | Ser | Ser | Ser | — | Ser | — | Ser | — | Ser | Ser | Ala |
| 2 | 4.5 | Ser | — | Ser | — | Ser | — | — | — | — | Ser | — |
| 3 | 4.7 | Ser | — | Ser | Ser | Ser | Ser | — | — | — | Ser | Ala |
| 4 | 5.5 | Ser | — | Ser | — | Ser | Ser | — | Ser | — | — | — |
| 5 | 5.7 | — | — | — | — | Ser | Ser | — | — | Ser | Ser | Ala |
| 6 | 5.8 | — | Ser | — | Ser | Ser | Ser | Ser | — | Ser | Tyr | Ala |
| 7 | 5.9 | Ser | — | Ser | — | Ser | Ser | — | — | Ser | — | Ala |
| 8 | 7.5 | Ser | — | — | Ser | Ser | Ser | — | Ser | Ser | — | Ser |
| 9 | 7.6 | — | — | — | Ser | Ser | Ser | — | — | — | Tyr | Ala |
| 10 | 7.7 | Ser | — | Ser | — | Ser | — | Ser | — | Ser | Ala | Ala |
| 11 | 7.8 | Ser | — | — | Ser | Ser | Ser | — | — | Ser | Tyr | Ser |
| 12 | 2.4 | — | Ser | — | — | Ser | Ser | — | — | — | Ala | Ala |
| 13 | 2.6 | Ser | — | Ser | — | Ser | Ser | — | Ser | Ser | Tyr | Ala |
| 14 | 2.9 | — | — | Ser | Ser | Ser | Ser | — | — | Ser | — | — |
| 15 | 3.1 | — | Ser | Ser | — | Ser | Ser | Ser | — | Ser | Tyr | — |
| 16 | 3.3 | Ser | Ser | — | — | Ser | Ser | Ser | — | — | Ser, | Ser |
| 17 | 3.7 | Ser | — | Ser | Ser | Ser | Ser | Ser | — | Ser | — | — |
| 18 | 4.3 | — | Ser | Ser | Ser | Ser | Ser | — | — | — | — | Ala |

FIGURE 7

EP 1 495 142 B1

☑ REQUIRED MUTATIONS only
● REQUIRED (mixed w/else) MUTATION DISTRIBUTION

| No. of SUBST. | REQUESTED MUTANTS | | REQ. MUT. MIXED W/ELSE | | ALL MUTANTS | |
|---|---|---|---|---|---|---|
| | probability | cumulative | probability | cumulative | probability | cumulative |
| 0 | 0.000000 | 0.0000 | 0.000000 | 0.0000 | 0.000977 | 0.0010 |
| 1 | 0.006836 | 0.0068 | 0.131836 | 0.1318 | 0.012695 | 0.0137 |
| 2 | 0.020508 | 0.0273 | 0.272461 | 0.4043 | 0.067383 | 0.0811 |
| 3 | 0.034180 | 0.0615 | 0.299805 | 0.7041 | 0.188477 | 0.2695 |
| 4 | 0.034180 | 0.0957 | 0.188477 | 0.8926 | 0.299805 | 0.5693 |
| 5 | 0.020508 | 0.1162 | 0.067383 | 0.9600 | 0.272461 | 0.8418 |
| 6 | 0.006836 | 0.1230 | 0.012695 | 0.9727 | 0.131836 | 0.9736 |
| 7 | 0.000977 | 0.1240 | 0.000977 | 0.9736 | 0.026367 | 1.0000 |

FIG. 8

☒ REQUIRED MUTATIONS ONLY
● REQUIRED (mixed w/else) MUTATION DISTRIBUTION

Probability (left axis): 0.35, 0.30, 0.25, 0.20, 0.15, 0.10, 0.05, 0

Cumulative Probability (right axis): 1.0, 0.8, 0.6, 0.4, 0.2, 0

Total No. of Positions Changed: 0, 2, 4, 6

| No. of SUBST. | REQUESTED MUTANTS | | REQ. MUT. MIXED W/ELSE | | ALL MUTANTS | |
|---|---|---|---|---|---|---|
| | probability | cumulative | probability | cumulative | probability | cumulative |
| 0 | 0.000000 | 0.0000 | 0.000000 | 0.0000 | 0.035959 | 0.0360 |
| 1 | 0.062929 | 0.0629 | 0.395553 | 0.3956 | 0.170807 | 0.2068 |
| 2 | 0.047197 | 0.1101 | 0.229241 | 0.6248 | 0.316892 | 0.5237 |
| 3 | 0.019665 | 0.1298 | 0.072481 | 0.6973 | 0.291608 | 0.8153 |
| 4 | 0.004916 | 0.1347 | 0.013485 | 0.7108 | 0.142012 | 0.9573 |
| 5 | 0.000737 | 0.1354 | 0.001475 | 0.7122 | 0.037399 | 0.9947 |
| 6 | 0.000061 | 0.1355 | 0.000088 | 0.7123 | 0.005048 | 0.9997 |
| 7 | 0.000002 | 0.1355 | 0.000002 | 0.7123 | 0.000274 | 1.0000 |

FIG. 9

EP 1 495 142 B1

REQUIRED MUTATIONS ONLY
REQUIRED (mixed w/else) MUTATION DISTRIBUTION

| No. of SUBST. | REQUESTED MUTANTS | | REQ. MUT. MIXED W/ELSE | | ALL MUTANTS | |
|---|---|---|---|---|---|---|
| | probability | cumulative | probability | cumulative | probability | cumulative |
| 0 | 0.000000 | 0.0000 | 0.000000 | 0.0000 | 0.116772 | 0.1168 |
| 1 | 0.090822 | 0.0908 | 0.348393 | 0.3484 | 0.324366 | 0.4411 |
| 2 | 0.030274 | 0.1211 | 0.095307 | 0.4437 | 0.341665 | 0.7828 |
| 3 | 0.005606 | 0.1267 | 0.014339 | 0.4580 | 0.169952 | 0.9528 |
| 4 | 0.000623 | 0.1273 | 0.001281 | 0.4593 | 0.041628 | 0.9944 |
| 5 | 0.000042 | 0.1274 | 0.000068 | 0.4594 | 0.005274 | 0.9997 |
| 6 | 0.000002 | 0.1274 | 0.000002 | 0.4594 | 0.000334 | 1.0000 |
| 7 | 0.000000 | 0.1274 | 0.000000 | 0.4594 | 0.000008 | 1.0000 |

FIG. 10

EP 1 495 142 B1

# CDR2

## *His walk-through, ratio 90:10*

| W.T. | 55 Gly | 56 Asn | 57 Lys | 58 Tyr | 59 Thr | 60 Thr | 61 Glu | 62 Tyr | 63 Ser | 64 Ala | 65 Ser | 66 Val | 67 Lys | 68 Gly |
|---|---|---|---|---|---|---|---|---|---|---|---|---|---|---|
| 1  2.0 | — | — | — | — | — | — | — | — | — | — | — | — | — | Asp |
| 2  2.1 | — | — | — | — | — | — | Asp | — | — | — | Pro | Asp | — | — |
| 3  2.3 | — | — | — | — | — | — | — | — | Arg | — | — | — | — | — |
| 4  2.4 | Arg | — | — | — | Asn | — | — | — | — | — | — | — | — | — |
| 5  2.6 | — | — | — | — | — | — | — | — | — | Asp | Pro | — | — | — |
| 6  2.7 | — | His | — | — | — | — | — | — | Arg | Asp | — | — | Arg | — |
| 7  2.8 | — | — | — | — | Asn | — | — | — | Arg | — | — | Leu | — | Arg |
| 8  3.0 | Arg | — | — | — | — | Pro | — | — | — | — | — | — | — | — |
| 9  3.1 | Asp | — | Asn | — | — | — | — | — | Asn | — | — | Leu | — | — |
| 10  3.3 | Asp | — | Asn | — | — | — | — | — | — | — | — | — | Asn | — |
| 11  3.4 | — | — | — | — | — | Asn | Gln | — | — | Pro | — | Asp | — | — |
| 12  3.5 | — | — | Asn | — | — | Asn | — | — | — | — | — | — | — | — |
| 13  3.6 | — | — | — | — | — | — | — | — | — | — | — | Asp | — | Asp |
| 14  3.7 | Asp | — | Asn | His | Pro | Asn | — | — | — | Pro | — | — | Asn | — |
| 15  3.8 | — | — | — | — | — | — | — | — | Asn | Pro | — | Asp | — | — |
| 16  3.9 | Asp | — | — | — | — | — | — | — | — | — | — | — | — | — |
| 17  4.0 | — | — | — | — | — | — | — | — | — | — | — | — | Gln | — |
| 18  4.1 | His | — | — | — | — | — | His | — | — | — | — | Leu | — | — |
| 19  4.3 | — | — | — | — | — | Asn | — | — | Asn | — | — | Asp | — | Arg |
| 20  4.4 | — | — | — | — | — | — | — | — | Arg | — | — | — | Gln | Asp |

FIGURE 11

EP 1 495 142 B1

FIG. 12

FIG. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9115581 A
- WO 0148485 A
- US 5830650 A
- US 5798208 A
- US 4888286 A
- US 373558 P
- US 0311935 W
- US 60373686 B

### Non-patent literature cited in the description

- **G. WEISS et al.** *PNAS,* 01 August 2000, vol. 97 (16), 8950-8954
- **J. HERMES et al.** *Gene,* 1989, vol. 84, 143-151
- **L. JENSEN et al.** *Nucleic Acids Research,* 1998, vol. 26 (3), 697-702
- **G. E. SCHULZ ; R. M. SCHIRNER.** Principles of Protein Structure. Springer-Verlag, 1988
- **HUSE, W. D et al.** *Science,* 1989, vol. 246, 1275
- **VIERA, J et al.** *Meth. Enzymol,* 1987, vol. 153, 3
- **PLUCKTHUN, A ; SKERRA, A.** *Meth. Enzymol,* 1989, vol. 178, 476-515
- **SKERRA, A et al.** *Biotechnology,* 1991, vol. 9, 23-278
- **BETTER, M ; HORWITZ, A.** *Meth. Enzymol,* 1989, vol. 178, 476
- Recombinant Antibodies. Springer Lab manual. Spriger-Verlag, 2001, 4-16
- Stabilization Strategies and Application of recombinant Fvs and Fv Fusion proteins. Springer Lab manual. Spriger-Verlag, 2001, 593-615
- **KABAT et al.** Sequences of proteins of Immunological Interest. *US Department Of Health and Human Services, Public Service, NIH,* 1991
- **WASSERFALLEN, A ; REKIK, M ; HARAYAMA, S.** *Biotechnology,* 1991, vol. 9, 296-298
- **RUDIKOFF, S ; POTTER, M.** *Biochemistry,* 1974, vol. 13, 4033
- **PLUCKTHUN, A et al.** *Cold Spring Harbor Symp. Quant. Biol,* 1987, vol. LII, 105-112
- **SKERRA, A et al.** *Biotechnology,* 1991, vol. 9, 273-278